(19)

Europäisches
Patentamt
European
Patent Office
Office européen
des brevets

(11) **EP 2 336 154 A2**

(12) **EUROPEAN PATENT APPLICATION**

(43) Date of publication:
 **22.06.2011 Bulletin 2011/25**

(21) Application number: **10180541.4**

(22) Date of filing: **19.11.2007**

(51) Int Cl.:
 *C07K 14/435* (2006.01)       *C12N 9/14* (2006.01)
 *C12N 15/12* (2006.01)

(84) Designated Contracting States:
 **AT BE BG CH CY CZ DE DK EE ES FI FR GB GR
 HU IE IS IT LI LT LU LV MC MT NL PL PT RO SE
 SI SK TR**

(30) Priority: **17.11.2006 JP 2006311241**

(62) Document number(s) of the earlier application(s) in
 accordance with Art. 76 EPC:
 **07832436.5 / 2 094 723**

(71) Applicant: **Sumitomo Chemical Company, Limited
 Tokyo 104-8260 (JP)**

(72) Inventors:
 • **Shimokawatoko, Yasutaka
  Hyogo 654-0131 (JP)**
 • **Van De Craen, Marc
  9880 Aalter (BE)**

 • **Nooren, Irene
  2341 PH Oegstgeest (NL)**
 • **Oosthuyse, Bert
  8710 Ooigem (BE)**
 • **Demey, Bert
  8770 Ingelmunster (BE)**
 • **Maddelein, Wendy
  9860 Gijzenzele (BE)**
 • **Drevon, Geraldine
  9000 Gent (BE)**

(74) Representative: **Vossius & Partner
 Siebertstrasse 4
 81675 München (DE)**

Remarks:
 This application was filed on 28-09-2010 as a
 divisional application to the application mentioned
 under INID code 62.

(54) **Agent that modulates physiological condition of pests, involved in insect vesicle-fusing
 atpase activity**

(57)  The present invention provides an agent that modulates physiological condition of pests, wherein the agent has an ability to modulate the activity of an insect vesicle-fusing ATPase; a method for assaying pesticidal activity of a test substance, which comprises measuring the activity of a vesicle-fusing ATPase in a reaction system in which the vesicle-fusing ATPase contacts with a test substance, and the like.

EP 2 336 154 A2

SEQUENCE LISTING

<110> Sumitomo Chemical Co., Ltd.

<120> AGENT THAT MODULATES PHYSIOLOGICAL CONDITION OF PESTS, INVOLVED IN INSECT VESICLE-FUSING ATPASE ACTIVITY

<130> S16999W001

<150> JP 2006/311241

<151> 2006-11-17

<160> 6

<210> 1

<211> 747

<212> PRT

<213> Aphis gossypii

<400> 1

Met Val Val Tyr Lys Ala Ile Lys Cys Leu Ala Asp Asp Trp Ala Leu
1               5                   10                  15

Thr Asn Cys Ala Val Val Asn Asp Glu Asp Phe Arg Ser Asp Thr Lys
            20                  25                  30

Tyr Ile Glu Val Gln His Gln Leu Lys Pro Asp Leu Lys Phe Trp Phe
        35                  40                  45

Thr Ile Leu Phe Val Lys Asn Pro Pro Pro Arg Gly Cys Ile Ala Phe
          50                    55                    60

Ser Val Asn Gln Arg Glu Trp Ala Gln Leu Ser Ile Asn Gln Pro Ile
    65                    70                    75                    80

Ser Ile Thr Ser Tyr Pro Gly Asn Thr Ala Ile Asp Phe Leu Cys Ser
                  85                    90                    95

Val Glu Ala Glu Ile Asp Tyr Phe Gln Lys Asn Lys Ser Lys Ala Asn
              100                    105                    110

Glu Gln Phe Asp Thr Asp Met Met Ala Lys Glu Phe Leu Val Asn Phe
          115                    120                    125

Thr Asn His Val Leu Ser Val Ser Gln Thr Leu Leu Phe Gln Leu Pro
    130                    135                    140

Glu Lys Pro Leu Met Thr Ile Lys Ile Lys Ser Ile Glu Gly Val Asn
145                    150                    155                    160

Ser Gln Glu Ile Asn Ser Gly Ala Lys Pro Arg Ser Ile Gln Tyr Gly
                  165                    170                    175

Lys Cys Leu Ser Asn Thr Ala Val Arg Phe Val Val Gly Ser Asn Thr
              180                    185                    190

Ser Leu Met Leu Val Gly Lys Ser Lys Cys Gln Gln Ala Arg Val Ser
            195                 200                 205

Ile Ile Asn Pro Asp Phe Asp Phe Asn Lys Met Gly Ile Gly Gly Leu
            210                 215                 220

Asp Thr Glu Phe Asn Ala Ile Phe Arg Arg Ala Phe Ala Ser Arg Val
225                 230                 235                 240

Phe Pro Gln Glu Ile Ile Glu Gln Leu Gly Cys Lys His Val Lys Gly
                245                 250                 255

Ile Leu Leu Tyr Gly Pro Pro Gly Thr Gly Lys Thr Leu Met Ala Arg
                260                 265                 270

Gln Ile Gly Gln Met Leu Asn Ala Arg Glu Pro Lys Ile Val Asn Gly
                275                 280                 285

Pro Gln Ile Leu Asp Lys Tyr Val Gly Glu Ser Glu Ala Asn Ile Arg
            290                 295                 300

Arg Leu Phe Ala Asp Ala Glu Glu Glu Glu Lys Lys Ser Gly Ser Ala
305                 310                 315                 320

Ser Gly Leu His Ile Ile Ile Phe Asp Glu Ile Asp Ala Ile Cys Lys
                325                 330                 335

Ala Arg Gly Ser Val Gly Gly Asn Thr Gly Val His Asp Thr Val Val

340  345  350

Asn Gln Leu Leu Ala Lys Ile Asp Gly Val Glu Gln Leu Asn Asn Ile
355  360  365

Leu Val Ile Gly Met Thr Asn Arg Arg Asp Met Ile Asp Glu Ala Leu
370  375  380

Leu Arg Pro Gly Arg Leu Glu Val Gln Met Glu Ile Ser Leu Pro Asp
385  390  395  400

Glu His Gly Arg His Gln Ile Leu Asn Ile His Thr Thr Arg Met Lys
405  410  415

Glu Phe Lys Lys Ile Ala Asp Asp Val Asp Met Lys Glu Leu Ser Ile
420  425  430

Arg Thr Lys Asn Phe Ser Gly Ala Glu Leu Glu Gly Leu Val Arg Ala
435  440  445

Ala Gln Ser Thr Ala Met Asn Arg Leu Ile Lys Ala Asn Asn Lys Val
450  455  460

Glu Val Asp Pro Asp Ala Ser Glu Lys Leu Gln Val Cys Lys Glu Asp
465  470  475  480

Phe Leu His Ala Leu Glu Tyr Asp Ile Lys Pro Ala Phe Gly Ala Ser
485  490  495

Ala Glu Ala Leu Glu His Phe Leu Ala Arg Gly Ile Ile Thr Trp Gly
500 505 510

Pro Ser Val Ser Gly Ile Leu Glu Asp Gly Thr Leu Leu Thr Gln Gln
515 520 525

Ala Arg Val Ala Asp Thr Phe Gly Leu Val Ser Val Leu Ile Glu Gly
530 535 540

Pro Pro Asn Ser Gly Lys Thr Ala Leu Ala Ala Lys Leu Ala Lys Asp
545 550 555 560

Ser Asp Phe Pro Phe Val Lys Val Cys Ser Pro Glu Asp Met Val Gly
565 570 575

Phe Thr Glu Thr Ala Lys Cys Leu Gln Ile Arg Lys Ile Phe Asp Asp
580 585 590

Ala Tyr Arg Ser Gln Leu Ser Cys Ile Leu Val Asp Asn Ile Glu Arg
595 600 605

Leu Leu Asp Tyr Gly Ser Ile Gly Pro Arg Tyr Ser Asn Leu Thr Leu
610 615 620

Gln Ala Leu Leu Val Leu Leu Lys Lys Gln Pro Pro Lys Gly Lys Lys
625 630 635 640

Leu Leu Val Leu Cys Thr Ser Ser Arg Lys Gln Val Leu Glu Glu Met
645 650 655

Glu Met Leu Ser Ala Phe Thr Ala Val Leu His Val Pro Asn Leu Ser
660 665 670

Gln Pro Glu Glu Leu Ile Thr Val Leu Glu Gln Phe Asp Leu Phe Thr
675 680 685

Lys Gln Asp Ile His Lys Ile Tyr Asn Gln Ile Ser Gly His Asn Val
690 695 700

Phe Ile Gly Ile Lys Lys Leu Leu Ala Leu Ile Asp Met Ala Arg Gln
705 710 715 720

Thr Asp Pro Lys Val Arg Val Ile Lys Phe Leu Thr Lys Met Glu Glu
725 730 735

Glu Gly Cys Leu Asp Leu Gly Thr Met Ile His
740 745

<210> 2

<211> 2241

<212> DNA

<213> Aphis gossypii

<220>

<221> CDS

<222> (1)..(2241)

<400> 2

atg gtg gtt tac aag gca atc aaa tgt ttg gca gac gat tgg gct ctt    48
Met Val Val Tyr Lys Ala Ile Lys Cys Leu Ala Asp Asp Trp Ala Leu
 1               5                   10                  15

acg aat tgt gca gtg gtt aat gat gaa gac ttt cga agc gat aca aaa    96
Thr Asn Cys Ala Val Val Asn Asp Glu Asp Phe Arg Ser Asp Thr Lys
                 20                  25                  30

tac ata gag gtc caa cat caa ctc aaa cca gat ctt aag ttt tgg ttt   144
Tyr Ile Glu Val Gln His Gln Leu Lys Pro Asp Leu Lys Phe Trp Phe
             35                  40                  45

aca ata ttg ttt gtg aaa aat cct cct cca cga gga tgc ata gct ttt   192
Thr Ile Leu Phe Val Lys Asn Pro Pro Pro Arg Gly Cys Ile Ala Phe
         50                  55                  60

tct gtg aat caa cga gaa tgg gcc caa ctg tct atc aat cag cca atc   240
Ser Val Asn Gln Arg Glu Trp Ala Gln Leu Ser Ile Asn Gln Pro Ile
 65                  70                  75                  80

agt att act tct tat ccg ggc aat aca gct atc gat ttt ttg tgt tca   288
Ser Ile Thr Ser Tyr Pro Gly Asn Thr Ala Ile Asp Phe Leu Cys Ser
                 85                  90                  95

gtt gaa gct gaa att gac tat ttt caa aag aat aag tca aaa gct aat   336

Val Glu Ala Glu Ile Asp Tyr Phe Gln Lys Asn Lys Ser Lys Ala Asn
          100                 105                 110


gaa cag ttt gat act gac atg atg gct aaa gag ttt tta gta aat ttc    384
Glu Gln Phe Asp Thr Asp Met Met Ala Lys Glu Phe Leu Val Asn Phe
          115                 120                 125


aca aat cac gtt tta tct gtc agt cag aca tta tta ttt cag tta cca    432
Thr Asn His Val Leu Ser Val Ser Gln Thr Leu Leu Phe Gln Leu Pro
          130                 135                 140


gaa aaa cca tta atg aca att aag atc aaa agc att gaa ggt gta aat    480
Glu Lys Pro Leu Met Thr Ile Lys Ile Lys Ser Ile Glu Gly Val Asn
145                 150                 155                 160


tct caa gaa att aat tca gga gca aaa ccc cgg tct att caa tat ggg    528
Ser Gln Glu Ile Asn Ser Gly Ala Lys Pro Arg Ser Ile Gln Tyr Gly
          165                 170                 175


aaa tgt ttg agc aat aca gct gta cga ttt gta gtc ggc tcc aat act    576
Lys Cys Leu Ser Asn Thr Ala Val Arg Phe Val Val Gly Ser Asn Thr
          180                 185                 190


tca tta atg ttg gtc gga aag tca aaa tgc caa caa gca cgt gta tca    624
Ser Leu Met Leu Val Gly Lys Ser Lys Cys Gln Gln Ala Arg Val Ser
          195                 200                 205


att ata aat cca gat ttt gac ttc aat aaa atg gga att ggt ggt ttg    672

Ile Ile Asn Pro Asp Phe Asp Phe Asn Lys Met Gly Ile Gly Gly Leu
        210                 215                 220

gat aca gag ttt aat gct att ttt aga aga gca ttt gca tct aga gta    720
Asp Thr Glu Phe Asn Ala Ile Phe Arg Arg Ala Phe Ala Ser Arg Val
225                 230                 235                 240

ttt cca caa gag ata att gaa caa ctt ggg tgc aaa cac gtc aaa ggt    768
Phe Pro Gln Glu Ile Ile Glu Gln Leu Gly Cys Lys His Val Lys Gly
                    245                 250                 255

att ttg ctt tat ggt cca cct gga act ggt aaa aca ttg atg gct aga    816
Ile Leu Leu Tyr Gly Pro Pro Gly Thr Gly Lys Thr Leu Met Ala Arg
                260                 265                 270

caa att gga caa atg tta aat gca cgt gaa ccc aaa att gtt aat ggc    864
Gln Ile Gly Gln Met Leu Asn Ala Arg Glu Pro Lys Ile Val Asn Gly
            275                 280                 285

ccg caa att ttg gat aaa tat gtt ggt gaa tct gaa gca aat ata aga    912
Pro Gln Ile Leu Asp Lys Tyr Val Gly Glu Ser Glu Ala Asn Ile Arg
        290                 295                 300

agg tta ttc gca gat gct gaa gaa gaa gaa aag aaa tct ggt tca gct    960
Arg Leu Phe Ala Asp Ala Glu Glu Glu Glu Lys Lys Ser Gly Ser Ala
305                 310                 315                 320

agt ggc tta cat ata att atc ttt gat gaa att gat gct att tgt aaa    1008

Ser Gly Leu His Ile Ile Ile Phe Asp Glu Ile Asp Ala Ile Cys Lys
325                     330                     335

gca aga ggt tct gtt gga gga aat aca gga gta cac gac act gta gtt    1056
Ala Arg Gly Ser Val Gly Gly Asn Thr Gly Val His Asp Thr Val Val
340                     345                     350

aat caa ttg tta gct aaa att gat gga gtc gag caa cta aat aac atc    1104
Asn Gln Leu Leu Ala Lys Ile Asp Gly Val Glu Gln Leu Asn Asn Ile
355                     360                     365

tta gtt ata ggt atg act aat aga aga gac atg att gat gaa gcg tta    1152
Leu Val Ile Gly Met Thr Asn Arg Arg Asp Met Ile Asp Glu Ala Leu
370                     375                     380

ctg aga cct ggc cga ctt gaa gta caa atg gaa ata agt tta cct gat    1200
Leu Arg Pro Gly Arg Leu Glu Val Gln Met Glu Ile Ser Leu Pro Asp
385                 390                     395                 400

gaa cat ggt cgt cat caa att ttg aat atc cac aca aca cgt atg aaa    1248
Glu His Gly Arg His Gln Ile Leu Asn Ile His Thr Thr Arg Met Lys
405                     410                     415

gaa ttt aaa aag att gct gat gat gta gac atg aag gaa ctt tca ata    1296
Glu Phe Lys Lys Ile Ala Asp Asp Val Asp Met Lys Glu Leu Ser Ile
420                     425                     430

cga act aaa aac ttc agt ggt gcc gag ttg gaa ggt tta gtt cgt gct    1344

Arg Thr Lys Asn Phe Ser Gly Ala Glu Leu Glu Gly Leu Val Arg Ala

     435              440              445

gct caa tca aca gcc atg aat cgt ttg ata aaa gcc aat aat aag gta    1392

Ala Gln Ser Thr Ala Met Asn Arg Leu Ile Lys Ala Asn Asn Lys Val

     450              455              460

gaa gtt gat cct gat gca tct gaa aaa ctt caa gtg tgc aag gaa gat    1440

Glu Val Asp Pro Asp Ala Ser Glu Lys Leu Gln Val Cys Lys Glu Asp

465           470              475              480

ttt cta cat gca ttg gaa tat gat ata aaa ccc gcg ttt ggt gct agt    1488

Phe Leu His Ala Leu Glu Tyr Asp Ile Lys Pro Ala Phe Gly Ala Ser

         485              490              495

gct gaa gct ttg gaa cac ttc tta gct cgt ggt att ata act tgg ggt    1536

Ala Glu Ala Leu Glu His Phe Leu Ala Arg Gly Ile Ile Thr Trp Gly

         500              505              510

ccg tct gtc agt gga att tta gaa gat gga aca ttg ctt aca cag caa    1584

Pro Ser Val Ser Gly Ile Leu Glu Asp Gly Thr Leu Leu Thr Gln Gln

         515              520              525

gct cgt gta gct gat aca ttt ggc ctt gtc tct gta ctc att gaa gga    1632

Ala Arg Val Ala Asp Thr Phe Gly Leu Val Ser Val Leu Ile Glu Gly

         530              535              540

ccg cca aat tca gga aaa act gct cta gct gca aag ttg gct aaa gat    1680

Pro Pro Asn Ser Gly Lys Thr Ala Leu Ala Ala Lys Leu Ala Lys Asp
545                550                555                560

tca gat ttc ccc ttt gtc aaa gtg tgt tca cca gaa gat atg gtt gga    1728
Ser Asp Phe Pro Phe Val Lys Val Cys Ser Pro Glu Asp Met Val Gly
               565                570                575

ttc act gaa aca gca aaa tgc tta caa atc aga aaa atc ttt gat gat    1776
Phe Thr Glu Thr Ala Lys Cys Leu Gln Ile Arg Lys Ile Phe Asp Asp
               580                585                590

gca tac aga tct caa ctt agt tgt att tta gtt gat aat att gaa cgt    1824
Ala Tyr Arg Ser Gln Leu Ser Cys Ile Leu Val Asp Asn Ile Glu Arg
          595                600                605

tta tta gac tat ggg tca att gga cca aga tat tct aac tta aca tta    1872
Leu Leu Asp Tyr Gly Ser Ile Gly Pro Arg Tyr Ser Asn Leu Thr Leu
     610                615                620

caa gct cta ttg gtt ctg tta aaa aaa caa ccc cct aaa ggt aaa aag    1920
Gln Ala Leu Leu Val Leu Leu Lys Lys Gln Pro Pro Lys Gly Lys Lys
625                630                635                640

ctt tta gta ttg tgt acc agc agt cgt aaa caa gta cta gaa gaa atg    1968
Leu Leu Val Leu Cys Thr Ser Ser Arg Lys Gln Val Leu Glu Glu Met
               645                650                655

gag atg tta tct gca ttt act gct gta ctt cat gtc cct aat ctc agt    2016

Glu Met Leu Ser Ala Phe Thr Ala Val Leu His Val Pro Asn Leu Ser
        660                     665                 670

caa cca gaa gaa ctt att acg gtt cta gaa caa ttt gat ttg ttt aca     2064
Gln Pro Glu Glu Leu Ile Thr Val Leu Glu Gln Phe Asp Leu Phe Thr
        675                     680                 685

aaa caa gat atc cac aag ata tac aac caa ata tct gga cac aat gtt     2112
Lys Gln Asp Ile His Lys Ile Tyr Asn Gln Ile Ser Gly His Asn Val
        690                     695                 700

ttt att ggt ata aaa aag ctg ttg gct ttg att gat atg gcc cgt caa     2160
Phe Ile Gly Ile Lys Lys Leu Leu Ala Leu Ile Asp Met Ala Arg Gln
705                 710                 715                 720

aca gat cca aaa gta aga gtg att aaa ttc tta act aag atg gaa gaa     2208
Thr Asp Pro Lys Val Arg Val Ile Lys Phe Leu Thr Lys Met Glu Glu
                725                 730                 735

gaa ggc tgt cta gat tta ggt act atg ata cat                         2241
Glu Gly Cys Leu Asp Leu Gly Thr Met Ile His
                740                 745

<210> 3

<211> 32

<212> DNA

<213> Artificial Sequence

42

<220>

<223> Description of Artificial Sequence:Designed oligonucleotide primer for PCR

<400> 3

cgttcggtag tggtgtccta ctaatgtaat tg        32

<210> 4

<211> 32

<212> DNA

<213> Artificial Sequence

<220>

<223> Description of Artificial Sequence:Designed oligonucleotide primer for PCR

<400> 4

catccgttga ctcttaacta atttcggaat gt        32

<210> 5

<211> 28

<212> DNA

<213> Artificial Sequence

<220>

<223> Description of Artificial Sequence:Designed oligonucleotide primer for PCR

<400> 5

atggtggttt acaaggcaat caaatgtt        28

<210> 6

<211> 33

<212> DNA

<213> Artificial Sequence

<220>

<223> Description of Artificial Sequence:Designed oligonucleotide primer for PCR

<400> 6

ctcgagcttg cacacttgaa gtttttcaga tgc        33

**Description**

Technical Field

The present invention relates to an agent that modulates physiological condition of pests, involved in insect vesicle-fusing ATPase activity, and the like.

Background Art

A vesicle-fusing ATPase is called an N5F (N-ethylmaleimide sensitive factor), belongs to the AAA (ATPase associated with various cellular activities) subgroup of P-loop ATPase (Chene, Nature Reviews Drug Discovery, 1: 665-673, 2002), and plays an important role in cellular vesicule trafficking. In addition, at least in vertebrates, a vesicle-fusing ATPase binds to and regulates the function and subcellular distribution of the AMPA class glutamate receptor (Hanley et al., Neuron, 34:53-67, 2002) .

Being common to vertebrates and invertebrates, the most characteristic function of vesicle-fusing ATPase is an involvement in a vesicle transport (Mayet al. , J. Biol.Chem., 276:21991-21994. 2001). During vesicle transport, vesicles fuse to a target membrane (for example, a synaptic membrane) and releases vesiclular content (for example, neurotransmitter) across the target membrane. Vesicle fusion is driven by the formation of tight-binding SNARE (soluble NSF attachment protein receptor) complex of proteins. After vesicle fusion, the vesicle-fusing ATPase hexamer binds to the SNARE complex and catalyzes its disassembly using the free energy from ATP hydrolysis, thus allowing recycling of the SNARE proteins for further rounds of membrane fusion. The vesicle-fusing ATPase is able to bind the SNARE complex only in the presence of the adaptor protein a-SNAP (soluble NSF attachment protein).

In contrast to C. elegans and human, there are two vesicle-fusing ATPase genes in D. melanogaster (nsfl[comt, comatose] and nsf2). Nsf1 encodes 745 amino acid residues and nsf2 encodes 752 amino acid residues, which show 84% amino acid identity to each other. It has been proposed that nsf1 has a pre-synaptic role (i.e., SNARE complex disassembly) and nsf2 has a post-synaptic role (i.e., glutamate receptor regulation) (Golby et al., Genetics, 158:265-278, 2001). On the other hand, ectopic expression experiments in D. melanogaster demonstrated that the nsf1 and nsf2 gene products could functionally substitute for one another (Golby et al., Genetics, 158:265-278, 2001). In insects NSF is also co-localized with two crucial neurohormones, prothoracicotropic hormone and diapause hormone, both of which regulate insect development. These findings suggest that NSF may be involved in regulating insect neurohormone release (Cui and Xu, Peptides, 27(6):1226-1234, 2006, and Cui et al., Insect Biochem Mol Biol., 36(7):603-9, 2006).

Loss of nsf -1 in C. elegans by RNAi leads to embryonic lethal, larval lethal, larval arrest, sterile and Membranous Organelles defective phenotypes (WormBase, http://www.wormbase.org/).

Analysis of nsf1 and nsf2 genetic mutations in D. melanogaster indicate that nsf1 is required for viability at the embryonic, larval and adult stages of development (WormBase, http://www.wormbase.org/), whereas nsf2 is required for viability at the first instar larval stage of development (Golby et al., Genetics, 158:265-278, 2001). Nsf1 probably functions primarily in a presynaptic capacity to regulate neurotransmitter release and nsf2 probably functions in a post-synaptic capacity.

A study of 16 recessive alleles of the X-linked comatose gene (nsf1) in D. melanogaster showed that hypomorphic mutations in nsf1 caused temperature-sensitive paralysis in adults, whereas null mutations resulted in embryonic and larval lethality (Littleton et al., Proc. Natl. Acad. Sci. USA, 98:12233-12238, 2001). All but one of the nine temperature-sensitive paralytic mutants altered highly conserved amino acids within the NSF-D1 domain, which is the domain exercising the ATPase activity that provides the driving force for a-SNAP-SNARE complex disassembly. Most of these mutants paralyzed within several minutes at 38°C, and showed a dramatic accumulation of SNARE complexes and blocked vesicle trafficking after heat-shock. Null mutants could be rescued by a heat-shock driven nsf1 transgene. Further analysis of one of the conditional paralytic alleles in combination with mutations in other genes known to block specific stages in vesicle trafficking indicated that NSF-1 normally dissociates and recycles SNARE proteins during the interval between exocytosis and endocytosis.

The nsf2 recessive lethal alleles of the chromosome-3 linked nsf2 gene appeared to develop normally as embryos, were able to hatch from the egg case, and showed normal larval locomotion as hemizygotes with an nsf2-deficient chromosome (Golby et al., Genetics, 158:265-278, 2001). However, the most severe nsf2 mutants ceased moving and died during the mid-first instar larval stage of development. All phenotypes could be rescued by expression of wild-type nsf2 under heat-shock control (heating at 38°C for 1 hour per day during development). Interestingly, ectopic expression of wild-type nsf2 under the control of a neuronal tissue-specific promoter failed to rescue different allelic mutants. In contrast, ectopic expression of wild-type nsf2 in mesoderm, which gives rise to somatic and visceral muscle, was sufficient to produce viable adults. The extent of rescue was higher in flies expressing nsf2 in both nervous system and mesoderm. These results suggest that NSF2 functions primarily in mesoderm but also has a secondary role in the nervous system.

Discovery of agricultural chemicals has traditionally been based on a random screening process, often directly testing the effects of specific chemicals on whole organisms, such as insects, fungi and/or plants and determining biological activity. Once chemical compounds with the appropriate biological activity are discovered, more intense research is required to specifically determine the mode of action or site of action of these compounds at the molecular level, in order to predict safety and environmental load of these compounds.

Disclosure of Invention

This invention describes a more target-based approach of screening agricultural chemicals, whereby compounds are screened against a specific target that has been identified as biologically and/or physiologically relevant with intent of chemically interfering with the target site to control insects or other pest organism.

Specifically, this invention describes that an agent that modulates physiological condition of pests and having an ability to modulate the activity of an insect vesicle-fusing ATPase is useful to control pests.

That is, the present invention provides:

1. An agent that modulates physiological condition of pests, wherein said agent has an ability to modulate the activity of an insect vesicle-fusing ATPase;

2. An agent according to item 1, wherein said vesicle-fusing ATPase is a cotton aphid vesicle-fusing ATPase;

3. An agent according to item I, wherein said agent is a pesticidal agent;

4. An agent according to item 1, wherein said ability to modulate the activity of an insect vesicle-fusing ATPase is an ability to inhibit a reaction of the insect vesicle-fusing ATPase with ATP;

5. A pesticidal agent which comprises a substance that has an ability to modulate the activity of an insect vesicle-fusing ATPase or an agriculturally acceptable salt of the substance as an active ingredient;

6. A pesticidal agent according to item 5, wherein said substance has an ability to inhibit a reaction of the insect vesicle-fusing ATPase with ATP;

7. A pesticidal agent according to item 6, wherein said substance has an ability to inhibit the reaction of the insect vesicle-fusing ATPase with ATP in a cell-free system, wherein in the presence of said substance of 10 micro M or more the activity of said vesicle-fusing ATPase is lower than that in the absence of said substance;

8. A pesticidal agent according to item 6, wherein said substance has an ability to inhibit a reaction of the insect vesicle-fusing ATPase with ATP in a cell-free system with an IC50 of 100 micro M or less;

9. A method for assaying pesticidal activity of a test substance, which comprises:

(1) a first step of measuring the activity of a vesicle-fusing ATPase selected from the following group A in a reaction system in which said vesicle-fusing ATPase contacts with a test substance; and

(2) a second step of evaluating the pesticidal activity of said test substance based on the difference obtained by comparing the activity measured in the first step with the activity of a control:

<group A>

(a) a protein comprising the amino acid sequence of SEQ ID NO: 1;

(b) a protein comprising an amino acid sequence with deletion, addition or substitution of one or more amino acids in the amino acid sequence of SEQ ID NO: 1, wherein said protein has vesicle-fusing ATPase activity;

(c) a protein comprising an amino acid sequence that has sequence identity of 75% or more to the amino acid sequence of SEQ ID NO: 1, wherein said protein has vesicle-fusing ATPase activity;

(d) a protein comprising the amino acid sequence encoded by the nucleotide sequence of SEQ ID NO: 2;

(e) a protein comprising an amino acid sequence encoded by a nucleotide sequence that has sequence identity of 75% or more to the nucleotide sequence of SEQ ID NO: 2, wherein said protein has vesicle-fusing ATPase activity;

(f) a protein comprising an amino acid sequence encoded by a polynucleotide, wherein said polynucleotide hybridizes under a stringent condition to a polynucleotide comprising a nucleotide sequence complementary to the nucleotide sequence of SEQ ID NO: 2, and wherein said protein has vesicle-fusing ATPase activity;

(g) a protein comprising an amino acid sequence of an insect vesicle-fusing ATPase; and

(h) a protein comprising an amino acid sequence of a cotton aphid vesicle-fusing ATPase;

10. A method for screening a pesticidal substance, which comprises selecting a substance having the pesticidal activity that is evaluated by the method of item 9;

11. A pesticidal agent which comprises a substance selected by the method of item 10 or agriculturally acceptable salts thereof as an active ingredient;

12. A method for controlling pests which comprises applying an effective amount of the pesticidal agent of item 5, 6,7,8 or 11 to the pest, habitat of the pest or plant to be protected from the pest;

13. A method for controlling pests which comprises:

identifying a substance having the pesticidal activity that is evaluated by the method of item 9, and
contacting the pest with the identified pesticidal substance;

14. An insect vesicle-fusing ATPase comprising an amino acid sequence selected from the following group B:

<group B>

(a) the amino acid sequence of SEQ ID NO: 1;
(b) an amino acid sequence with deletion, addition or substitution of one or more amino acids in the amino acid sequence of SEQ ID NO: 1, wherein said amino acid sequence has vesicle-fusing ATPase activity;
(c) an amino acid sequence that has sequence identity of 75% or more to the amino acid sequence of SEQ ID NO: 1, wherein said amino acid sequence has vesicle-fusing ATPase activity;
(d) the amino acid sequence encoded by the nucleotide sequence of SEQ ID NO: 2;
(e) an amino acid sequence encoded by a nucleotide sequence that has sequence identity of 75% or more to the nucleotide sequence of SEQ ID NO: 2, wherein said amino acid sequence has vesicle-fusing ATPase activity;
(f) an amino acid sequence encoded by a polynucleotide, wherein said polynucleotide hybridizes under a stringent condition to a polynucleotide comprising a nucleotide sequence complementary to the nucleotide sequence of SEQ ID NO: 2, wherein said amino acid sequence has vesicle-fusing ATPase activity; and
(g) an amino acid sequence of a cotton aphid vesicle-fusing ATPase;

15. Use of an insect vesicle-fusing ATPase as a reagent that provides an indicator to evaluate pesticidal activity;

16. Use of an insect vesicle-fusing ATPase of item 14 as a reagent that provides an indicator to evaluate pesticidal activity;

17. A polynucleotide which comprises a nucleotide sequence encoding an amino acid sequence of a vesicle-fusing ATPase of item 14;

18. A polynucleotide according to item 17, which comprises the nucleotide sequence of SEQ ID NO: 2;

19. A polynucleotide which comprises a nucleotide sequence complementary to a nucleotide sequence of a polynucleotide of item 17 or 18;

20. A polynucleotide which comprises:

a partial nucleotide sequence of a polynucleotide of item 17 or 18; or
a nucleotide sequence complementary to said partial nucleotide sequence;

21. A polynucleotide according to item 20, which comprises a nucleotide sequence of SEQ ID NO: 3 or 4;

22. A method for obtaining a polynucleotide comprising a nucleotide sequence encoding an amino acid sequence of a vesicle-fusing ATPase, which comprises:

amplifying a desired polynucleotide by polymerase chain reaction using as a primer a polynucleotide of item 20 or 21;
identifying the desired polynucleotide amplified; and
recovering the identified polynucleotide;

23. A method for obtaining a polynucleotide comprising a nucleotide sequence encoding an amino acid sequence of a vesicle-fusing ATPase, which comprises:

detecting a desired polynucleotide by hybridization using as a probe a polynucleotide of item 19, 20 or 21;
identifying the desired polynucleotide detected; and
recovering the identified polynucleotide;

24. A circular polynucleotide comprising a nucleotide sequence of a polynucleotide of item 17 or 18, wherein said nucleotide sequence is operably linked to a bacteriophage promoter;

25. A circular polynucleotide according to item 24, wherein said promoter is a T7 RNA polymerase gene promoter;

26. A circular polynucleotide according to item 24 or 25, wherein said polynucleotide comprises a replication origin for autonomous replication in a host cell;

27. A method for producing a circular polynucleotide, which comprises ligating a polynucleotides of item 17 or 18 into a vector;

28. A transformant in which a polynucleotide of item 17 or 18 is introduced;

29. A transformant according to item 28, wherein said transformant is a transformed E. coli;

30. A method for producing a transformant, which comprises introducing a polynucleotide of item 17 or 18 into a host cell;

31. A method for producing a vesicle-fusing ATPase, which comprises a step of culturing the transformant of item 28 or 29 and recovering a produced vesicle-fusing ATPase;

32. Use of a vesicle-fusing ATPase of item 14 or a polynucleotide of any one of items 17 to 21 as a research tool;

33. Use according to item 32, wherein the research tool is an experimental tool for screening a pesticidal substance; and

34. A system which comprises:

a means to input, store and manage a data information of an ability of test substances, wherein said ability is an ability to modulate the activity of an insect vesicle-fusing ATPase;

a means to query and retrieve the data information based on a desired criterion; and

a means to display and output the result which is queried and retrieved.

Modes for Carrying Out the Invention

The present invention will be explained in detail below.

In the present invention, the "pests" indicates small animals which cause harm or discomfort to life of the people by harming man and animals directly or by damaging crops. Examples thereof include arthropod such as insects, mites and ticks and Nematoda, and typical examples of which are as follows:

Hemiptera:

Delphacidae such as Laodelphax striatellus, Nilaparvata lugens and Sogatella furcifera, Deltocephalidae such as Nephotettix cincticeps and Empoasca onukii, Aphididae such as Aphis gossypii and Myzus persicae, Pentatomidae, Aleyrodidae such as Trialeurodes vaporariorum, Bemisia tabaci and Bemisia argentifolli, Coccidae, Tingidae, Psyllidae, etc.

Lepidoptera:

Pyralidae such as Chilo suppressalis, Cnaphalocrocis medinalis, Ostrinia nubilalis and Parapediasia teterrella, Noctuidae such as Spodoptera litura, Spodoptera exigua, Pseudaletia separata, Mamestra brassicae, Agrotis ipsilon, Trichoplusia spp., Heliothis spp., Helicoverpa spp. and Earias spp., Pieridae such as Pieris rapae crucivora, Tortricidae such as Adoxophyes orana fasciata, Grapholita molesta and Cydia pomonella, Carposinidae such as Carposina niponensis , Bucculatricidae such as Lyonetia clerkella, Gracillariidae such as Phyllonorycter ringoniella, Phyllocnistidae such as Phyllocnistis citrella, Yponomeutidae such as Plutella xylostella, Gelechiidae such as Pectinophora gossypiella, Arctiidae, Tineidae, etc.

Diptera:

Culex such as Culex pipiens pallens, Cules tritaeniorhynchus and Culex quinquefasciatus, Aedes such as Aedes aegypti and Aedes albopictus, Anopheles such as Anophelinae sinensis, Chironomidae, Muscidae such as Musca domestica and Muscina stabulans, Calliphoridae, Sarcophagidae, Fannia canicularis, Anthomyiidae such as Delia Platura and Delia antigua, Trypetidae, Drosophilidae, Psychodidae, Simuliidae, Tabanidae, Stomoxyidae, Agromyzidae, etc.

Coleoptera:

Diabrotica such as Diabrotica virgifera virgifera and Diabrotica undecimpunctata howardi, Scarabaeidae such as Anomala cuprea and Anomala rufocuprea, Curculionidae such as Sitophilus zeamais, Lissorphoptrus oryzophilus and Calosobruchys chinensis, Tenebrionidae such as Tenebrio molitor and Tribolium castaneum, Chrysomelidae such as Oulema oryzae, Aulacophora femoralis, Phyllotreta striolata and Leptinotarsa decemlineata, Anobiidae, Epilachna spp. such as Epilachna vigintioctopunctata, Lyctidae, Bostrychidae, Cerambycidae, Paederus fuiscipes, etc.

Thysanoptera:

Thripidae such as Thrips spp. including Thrips palmi, Frankliniella spp. including Frankliniella occidentalis and Sciltothrips spp. including Sciltothrips dorsalis, Phlaeothripidae, etc.

Hymenoptera:

Tenthredinidae, Formicidae, Vespidae, etc.

Dictyoptera:

Blattidae, Blattellidae, etc.

Orthoptera:

Adrididae, Gryllotalpidae etc.

Siphonaptera:

Pulex irritans, etc.

Anoplura:

Pediculus humanus capitis, etc.

Isoptera:

Termitidae, etc.

Acarina:

Tetranychidae such as Tetranychus urticae, Tetranychus kanzawai, Panonychus citri, Panonychus ulmi, and Oligonychus spp., Eriophyidae such as Aculops pelekassi and Aculus schlechtendali, Tarsonemidae such as Polyphagotarsonemus laths, Tenuipalpidae. Tuckerellidae, Ixodidae such as Haemaphysalis longicornis, Haemaphysalis flava, Dermacentor taiwanicus, Ixodes ovatus , Ixodes persulcatus and Boophilus microplus, Acaridae such as Tyrophagus putrescentiae, Dermanyssidae, Cheyletidae such as Dermatophagoides farinae and Dermatophagoides ptrenyssnus, such as Cheyletus eruditus, Cheyletus malaccensis and Cheyletus moorei, Dermanyssus spp., etc.

Nematodes:

Pratylenchus coffeae, Pratylenchus fallax, Heterodera glycines, Globodera rostochiensis, Meloidogyne hapla, Meloidogyne incognita, etc.

In the present invention, the "modulate physiological condition of pests" indicates changing condition such as various phenomena in a living body which are maintained for living in pests, for example, function such as aspiration, digestion, secretion, body liquid circulation, metabolism, neurotransmission and the like, or mechanism thereof into condition apart from usual conditions. Examples include changing condition by cessation of aspiration so that oxygen necessary for internal metabolism of pests is not supplied, and changing condition by cessation of function of neurotransmission of pests so that various movements of pests are ceased.

In the present invention, the "agent which modulates physiological condition of pests" is an agent which can modulate physiological condition of pests when being applied to pests.

In the present invention, the "insect vesicle-fusing ATPase" indicates a vesicle-fusing ATPase that occurs in insect, among vesicle-fusing ATPase present in various organisms. Herein, insect is an animal classified under Animalia, Arthropoda, Insecta, and examples of which include arthropod of the order Protura, Collembola, Diplura, Thysanura, Ephemeroptera, Odonata, Plecoptera, Grylloblattodea, Orthoptera, Phasmatodea, Dermaptera, Mantodea, Blattaria, Isoptera, Embioptera, Psocoptera, Mallophaga, Anoplura, Thysanoptera, Hemiptera, Neuroptera, Mecoptera, Trichoptera, Lepidoptera, Coleoptera, Diptera, Hymenoptera, Siphonaptera, Strepsiptera, and the like.

A vesicle-fusing ATPase (Vesicle-fusing ATPase, EC.3.6.4.6, the synonym: N-ethylmaleimide sensitive factor; NSF) is one of the ATPases called AAA (ATPase associated with various cellular activities), which has a function for disassembly of SNARE complex associated with a vesicle transport, using the free energy from ATP hydrolysis.

The vesicle-fusing ATPase activity may be measured by means of an in vitro assay. Numerous assays exist which can be used to determine the vesicle-fusing ATPase activity using radiolabeled and non-radiolabeled ATP as substrate of the enzyme. For example, a vesicle-fusing ATPase activity may be measured radioactively by using a radiolabeled ATP, for example as described in Tagaya et al., J Biol Chem 268, 1993, 2662-2666 by using [a-32P] ATP. As for the rest, as described in Mueller et al., Nature Cell Biol 1, 1999, 335-340; Peters et al. , EMBO J 9, 1990, 1757-1767, there is an example of measuring a vesicle-fusing ATPase activity based on detection of released [32Pi] by using [γ-32P] ATP as a substrate.

Similarly, as methods using for measuring a vesicle-fusing ATPase activity, there is an assay using a non-radiolabeled ATP. In such a method includes, but is not limited to measurement of products after cleavage of ATP which is a substrate and colorimeter quantitative analysis of the cleaved substrate. The measuring methods as described above are described in Lanzetta et al., Analytical Biochemistry 100, 95-97 (1979); Lill et al., Cell 60, 271-280(1990). In addition, the vesicle-fusing ATPase activity may be measured by using another enzymatic reaction coupled with an enzymatic reaction of vesicle-fusing ATPase. For example, after developing the enzymatic reaction of vesicle-fusing ATPase with the ATP as the substrate, an amount of ATP which was not consumed can be measured as an amount of luminescence of luciferase by means of using Kinase-Glo™ Luminescent Kinase Assay (manufactured by Promega). The increase of the amount of luminescence and the vesicle-fusing ATPase activity are inversely proportional.

As for the rest, it is also possible to detect the vesicle-fusing ATPase activity, for example by means of a standard method such as a biological method (an in vivo measuring process) in which the ATPase activity is measured.

Among such various methods for measuring activity of vesicle-fusing ATPase, a method for measuring activities of multi-sample vesicle-fusing ATPase mechanically and effectively desirably utilizes the above-described reaction of the vesicle-fusing ATPase coupled with another enzymatic reaction. A specific example includes a method comprising after an enzymatic reaction of the vesicle-fusing ATPase in which ATP is used as a substrate, measuring the amount of ATP which was not consumed as an amount of luminescence of luciferase using Kinase-Glo™ Luminescent Kinase Assay (manufactured by Promega) in accordance with the method described in the instruction attached to said kit, and determining the vesicle-fusing ATPase activity from this amount of luminescence.

In addition, a method for measuring activity of insect vesicle-fusing ATPase can be performed by a similar method to that described above.

Several amino acid sequences of vesicle-fusing ATPase have been identified in different insect species, for example, D. melanogaster (NSFI [comt, comatose] , accession No. NP_524877; NSF2, accession No. NP_788676), Manduca sexta (accession No. AAF18300), Helicoverpa zea (accession No. AA065962), Helicoverpa armigera (accession No. AAW58140), Aedes aegypti (accession No. AA083118), Anopheles gambiae (accession No. XP_307148), and the like, which can be found in public databases. Also, several nucleotide sequences of vesicle-fusing ATPase genes have been identified in different insect species, for example, D. melanogaster (nsf1 [comt, comatose], accession No. NM_080138; nsf2, accession No. NM_176499), Manduca sexta (accession No. AF118384), Helicoverpa zea (accession No. AY220909), Helicoverpa armigera (accession No. AY864803), Aedes aegypti (accession No. AY314995), Anopheles gambiae (accession No. XM_307148), Bombyx mori (accession No. AY864804), and the like, which can be found in public databases.

Here "a public database" includes, for example, databases disclosed by each databank such as GenBank, DDBL, and EMBL, in which anyone can use the data registered in said databases without any limitation. Information about base sequences registered in GenBank can be obtained, for example, by searching based on the aforementioned Accession No. given to each of genes by GenBank from a Web page of National Center for Biotechnology Information (URL; http://www.ncbi.nlm.nih.gov). In fact, it is officially acknowledged that the data registered in INSD should be eternally stored and disclosed as the scientific source material in "Handling of Registered Data of DDBL, EMBL and GenBank" (on May 23 to 24, 2002) established by the International Consultative Committee which is a consultative organization of the International Nucleotide Sequence Databases (INSD) constructed by the above 3 databanks. Thus, any person skilled in the art can collate, search or obtain all of the data disclosed by each databank such as GenBank, DDBL and EMBL, based on the Accession Nos.

[0026] In addition, according to the methods described below, an amino acid sequence of vesicle-fusing ATPase and a nucleotide sequence of vesicle-fusing ATPase gene can be identified from a cotton aphid. The identified amino acid sequence of cotton aphid vesicle-fusing ATPase is shown in SEQ ID NO: 1, and the nucleotide sequence of cotton aphid vesicle-fusing ATPase gene is shown in SEQ ID NO: 2.

Several amino acid sequences of vesicle-fusing ATPase have been identified in animals other than insect, for example, Ceanorhabditis elegans (accession No. NP_740892), Homo sapiens (accession No. NP_006169), and the like, which can be found in public databases. Also, several nucleotide sequences of vesicle-fusing ATPase genes have been identified in animals other than insects, for example, Ceanorhabditis elegans (accession No. NM_170902), Homo sapiens (accession No. NM_006178), and the like, which can be found in public databases.

Table 1 shows Sequence identity of the amino acid sequence of cotton aphid vesicle-fusing ATPase (SEQ ID NO: 1) and the nucleotide sequence of cotton aphid vesicle-fusing ATPase gene (SEQ ID NO: 2) with the sequence of vesicle-fusing ATPase and gene thereof found in other animals.

Table 1

| Origin of sequence | Identity of amino acid sequence (%) vs SEQ ID NO:1 | Identity of nucleotide sequence (%) vs SEQ ID NO:2 |
|---|---|---|
| D. melanogaster NSF1 | 67 | 64 |
| D. melanogaster NSF2 | 68 | 64 |
| Aedes aegypti | 70 | 67 |
| Apis mellifera | 69 | 71 |
| Manduca sexta | 70 | 65 |
| Helicoverpa armigera | 71 | 68 |
| Helicoverpa zea | 69 | 68 |
| Homo sapiens | 60 | 64 |
| Macaca mulata | 60 | 64 |
| Bos taurus | 60 | 64 |
| Canis familiaris | 60 | 64 |
| Mus musculus | 60 | 63 |
| Rattus norvegius | 60 | 63 |
| Cricetulus longicaudatus | 60 | 63 |
| Cricetulus griseus | 60 | - |
| Gallus gallus | 60 | 64 |
| Xenopus laevis | 59 | 62 |
| Danio renio | 59 | 63 |
| Ceanorhabditis elegans | 53 | 63 |
| Strongylocentrotus purpuratus | 57 | 64 |
| Theileria annulata | 42 | 60 |
| Entamoeba histolytica | 42 | 62 |
| Aspergillus niger | 46 | 58 |
| Toxoplasma gondii | 45 | 57 |
| Pichia pastoris | 46 | 62 |
| Saccharomyces cerevisiae | 45 | 61 |
| Schizosaccharomyces pombe | 49 | - |

An ability to modulate the activity of an insect vesicle-fusing ATPase refers to an ability to increase or decrease activity of an insect vesicle-fusing ATPase, that is, means an ability to activate a vesicle-fusing ATPase, or an ability to inhibit activity of a vesicle-fusing ATPase. And, a test substance can be added to the reaction system for measuring vesicle-fusing ATPase activity to investigate influence of the test substance on the vesicle-fusing ATPase activity.

Several ATPase inhibitors have been identified (Chene, Nature reviews, 1 (2002) 665: Beukers et al., aunyn-Schmied. Arch. Pharmacol. 351: 523-528, 1995). Some of these, such as N-ethylmaleimide, show also activity against vesicle-fusing ATPase (Steel G. J. et al., Biochemistry 38, 1999, 7764-7772 and Morgan A. et al., JBC 269(7), 1994, 29347-29350).

An $IC_{50}$ value of a test substance in the reaction means a concentration of a test substance at which 50% of the activity of the reaction with no test substance is inhibited. The $IC_{50}$ value of a test substance can be determined by adding test substances of different concentrations to the vesicle-fusing ATPase activity measuring reaction system, measuring the vesicle-fusing ATPase activity (response) at each concentration of added test substance(dose), producing a dose-response curve, and calculating a concentration of the added test substance, at which the vesicle-fusing ATPase activity is 50% inhibited. More specifically, a dose-response curve may be produced using 4 Parameter Logistic Model or Sigmoidal Dose-Response Model:

$$f(x) = (A+((B-A)/(1+((C/x)\hat{}D))))$$

$$f(x) = A + \frac{B-A}{1+(C/x)^D}$$

to calculate the $IC_{50}$. Practically, the $IC_{50}$ value may be calculated using XLfit (manufactured by IDBS) which is a commercially available calculating software.

An agent that has an ability to modulate the activity of an insect vesicle-fusing ATPase is an agent containing as an active ingredient a substance having an ability to modulate the activity of an insect vesicle-fusing ATPase.

In the present invention, the "agent that modulates physiological condition of pests, wherein the agent has an ability to modulate the activity of an insect vesicle-fusing ATPase" is an agent having an ability to modulate the activity of insect vesicle-fusing ATPase specified by the aforementioned measuring method, and means an agent that can modulate physiological condition of pests. Preferable examples of the agent include an agent in which an insect vesicle-fusing ATPase is a cotton aphid vesicle-fusing ATPase. In addition, preferable examples of the agent include an agent in which an agent that modulates physiological condition of pests is a pesticidal agent. In addition, preferable examples of the agent include an agent in which an ability to modulate the activity of an insect vesicle-fusing ATPase is an ability to inhibit a reaction using ATP as a substrate.

In the present invention, the "pesticidal agent" indicates an agent having an ability to control the pests.

Examples of a method for measuring an ability to control pests include, in addition to the methods disclosed in the present invention, a method of measuring pesticidal activity on the pests. Specifically, for example, the pesticidal activity can be measured according to the following method.

According to the method described in Handbook of Insect Rearing Vol.1 (Elsevier Science Publisers 1985), pp.35 to pp.36 except that a sterilized artificial feed having the following composition (Table 2) is prepared, and a solution of a test agent in DMSO is added at 0.5 % by volume of the artificial feed and is mixed, a cotton aphid is reared, the number of surviving cotton aphids is investigated after 6 days, and a controlling value is obtained according to the following equation.

Table 2

| Amino acid | (mg/100 ml) | Vitamins | (mg/100 ml) |
|---|---|---|---|
| L-Alanine | 100.0 | Ascorbic acid | 100.0 |
| L-arginine | 275.0 | Biotin | 0.1 |
| L-Asparagine | 550.0 | Calcium pantothenate | 5.0 |
| L-Aspartic acid | 140.0 | Choline chloride | 50.0 |
| L-cysteine (hydrochloride) | 40.0 | Inositol | 50.0 |
| L-glutamic acid | 140.0 | Nicotinic acid | 10.0 |
| L-glutamine | 150.0 | Thiamine | 2.5 |
| L-glycine | 80.0 | | |

(continued)

| Amino acid | (mg/100 ml) | Vitamins | (mg/100 ml) |
|---|---|---|---|
| L-histidine | 80.0 | Others | (mg/100 ml) |
| L-isoleucine | 80.0 | Sucrose | 12500.0 |
| L-leucine | 80.0 | Dipotassium hydrogen phosphate | 1500.0 |
| L-lysine (hydrochloride) | 120.0 | Magnesium sulfate | 123.0 |
| L-methionine | 80.0 | Cupric chloride | 0.2 |
| L-phenylalanine, | 40.0 | Ferric chloride | 11.0 |
| L-proline | 80.0 | Manganese chloride | 0.4 |
| L-serine | 80.0 | Zinc sulfate (anhydrous) | 0.8 |
| L-threonine | 140.0 | | |
| L-tryptophan | 80.0 | Adjusted to pH 6.8 | |
| L-tyrosine | 40.0 | | |
| L-valine | 80.0 | | |

$$\text{Controlling value (\%)} = \{1-(Cb \times Tai)/(Cai \times Tb)\} \times 100$$

Letters in the equation represent the following meanings.

Cb: Number of surviving worms before treatment in non-treating section
Cai: Number of surviving worms at observation in non-treated section
Tb: Number of surviving worms before treatment in non-treated section
Tai: Number of surviving worms at observation in a treated section

It may be said that a test agent exhibiting a significantly high controlling value has the pesticidal activity. More preferably, it may be determined that a test agent having the controlling value of 30% or more has substantial pesticidal activity, and it may be determined that a test agent having the controlling value of less than 30% has no substantial pesticidal activity.

The pesticidal agent in the present invention contains a chemical substance having an ability to modulate the activity of insect vesicle-fusing ATPase or an agriculturally acceptable salt thereof as an active ingredient.

In the present invention, an agriculturally acceptable salt refers to a salt in such a form that preparation of a controlling agent and application of the preparation do not become impossible, and may be a salt in any form. Specifically, examples of the salt include acid addition salts with mineral acids such as hydrochloric acid, hydrobromic acid, hydriodic acid, sulfuric acid, nitric acid, and phosphoric, organic acids such as formic acid, acetic acid, propionic acid, oxalic acid, malonic acid, succinic acid, fumaric acid, maleic acid, lactic acid, malic acid, tartaric acid, citric acid, methanesulfonic acid, and ethansulfonic acid, or acidic amino acids such as aspartic acid and glutamic acid; salts with inorganic bases such as sodium, potassium, magnesium, and aluminum, organic bases such as methylamine, ethylamine, and ethanolamine, or basic amino acids with lysine and ornithine; and an ammonium salts.

In the present invention, the "pesticidal agent which comprises a substance having an ability to modulate the activity of an insect vesicle-fusing ATPase or a an agriculturally acceptable salt thereof as an active ingredient" means an agent which can control pests by containing a substance having an ability to modulate the activity of insect vesicle-fusing ATPase identified in the measuring method or an agriculturally acceptable salt thereof as an active ingredient. Preferable examples of the substance include a compound having an ability to inhibit a reaction of a vesicle-fusing ATPase with ATP. More preferable examples of the substance include a substance having an ability to inhibit the reaction of the insect vesicle-fusing ATPase with ATP in a cell-free system, wherein in the presence of the substance of 10 $\mu$M or more the activity of the vesicle-fusing ATPase is lower than that in the absence of the substance. In addition, further preferable examples of the substance include a substance having an ability to inhibit a reaction of the insect vesicle-fusing ATPase with ATP in a cell-free system with an IC50 of 100 $\mu$M or less.

In the present invention, the "method for assaying pesticidal activity of a test substance, which comprises a first step of measuring the activity of a vesicle-fusing ATPase selected from the group A in a reaction system in which the vesicle-fusing ATPase contacts with a test substance, and a second step of evaluating the pestcidal activity of the test substance based on the difference obtained by comparing the activity measured in the first step with the activity of a control" indicates a method characterized by comprising the first step and the second step in various methods for assaying a pesticidal ability of a test substance. Herein, the group A indicates:

(a) a protein comprising the amino acid sequence of SEQ ID NO: 1;
(b) a protein comprising an amino acid sequence with deletion, addition or substitution of one or more amino acids in the amino acid sequence of SEQ ID NO: 1, wherein said protein has vesicle-fusing ATPase activity;
(c) a protein comprising an amino acid sequence that has sequence identity of 75% or more to the amino acid sequence of SEQ ID NO: 1, wherein said protein has vesicle-fusing ATPase activity;
(d) a protein comprising the amino acid sequence encoded by the nucleotide sequence of SEQ ID NO: 2;
(e) a protein comprising an amino acid sequence encoded by a nucleotide sequence that has sequence identity of 75% or more to the nucleotide sequence of SEQ ID NO: 2, wherein said protein has vesicle-fusing ATPase activity;
(f) a protein comprising an amino acid sequence encoded by a polynucleotide, wherein said polynucleotide hybridizes under a stringent condition to a polynucleotide comprising a nucleotide sequence complementary to the nucleotide sequence of SEQ ID NO: 2, and wherein said protein has vesicle-fusing ATPase activity;
(g) a protein comprising an amino acid sequence of an insect vesicle-fusing ATPase; and
(h) a protein comprising an amino acid sequence of a cotton aphid vesicle-fusing ATPase.

The first step is a step of measuring the activity of a vesicle-fusing ATPase in the state where a vesicle-fusing ATPase is contacted with a test substance by adding the test substance to the aforementioned various vesicle-fusing ATPase activity measuring reaction systems. In addition, the second step is a step of comparing the activity at measurement of a test substance with the substance of a control, and evaluating a pesticidal ability based on the difference. Herein, a control means, for example, in the case where a test substance dissolved in a solvent is added to the reaction system, a test section in which only a solvent same as that used to dissolve the test substance is added.

A vesicle-fusing ATPase used in a method for assaying a pesticidal ability possessed by a test substance, having the first step and the second step, is a protein shown in the group A. Among proteins of the group A, a difference which can be recognized between an amino acid sequence of protein represented by (a) and amino acid sequences of proteins represented by (b), (c), (e), (f), (g) and (h) is deletion, substitution, addition or the like of a part of amino acids. These include, for example, deletion due to processing which the protein having an amino acid sequence represented by (a) undergoes in a cell. In addition, examples include deletion, substitution, addition and the like of an amino acid generated by naturally occurring gene mutation due to a spices difference or an individual difference of an organism from which the protein is derived, or gene mutation which is artificially introduced by a site-directed mutagenesis, a random mutagenesis, mutation treatment or the like.

The number of amino acids undergoing the deletion, substitution, addition or the like may be the number in a range that the peptidase activity of a vesicle-fusing ATPase can be found out. In addition, examples of substitution of an amino acid include substitution with an amino acid which is similar in characteristic in hydrophobicity, charge, pH and steric structure. Specific examples of the substitution include substitution in an group of (1) glycine, alanine; (2) valine, isoleucine, leucine; (3) aspartic acid, glutamic acid, asparagine, glutamine, (4) serine, threonine; (5) lysine, arginine; (6) phenylalanine, tyrosine and the like.

Examples of a procedure of artificially introducing the deletion, addition or substitution of an amino acid (hereinafter, collectively referred to as alteration of amino acid in some cases) include a procedure of introducing site-directed mutation into a DNA encoding an amino acid sequence represented by (a) and, thereafter, expressing this DNA by a conventional method. Herein, examples of a site-directed mutagenesis include a method utilizing amber mutation (gapped duplex method, Nucleic Acids Res., 12, 9441-9456(1984)), a method by PCR using primers for mutation introduction, and the like. In addition, examples of a procedure of artificially altering an amino acid include a procedure of randomly introducing mutation into a DNA encoding an amino acid sequence represented by (a) and, thereafter, expressing this DNA by a conventional method. Herein, examples of a method of randomly introducing mutation include a method of performing PCR using a DNA encoding any of the aforementioned amino acid sequences as a template, and using a primer pair which can amplify each full length DNA at reaction condition under which an addition amount of each of dATP, dTTP, dGTP and dCTP used as a substrate is changed from a conventional concentration, or at reaction condition under which a concentration of $Mg^{2+}$ promoting a polymerase reaction is increased from a conventional concentration. Examples of the procedure of PCR include a method described, for example, in Method in Molecular Biology, (31), 1994, 97-112. Another example includes a method described in WO 0009682.

Herein, the "sequence identity" refers to identity between two nucleotide sequences or two amino acids. The "sequence identity" is determined by comparing two sequences which are aligned in the optimal state over an all region of sequences to be compared. Herein, in optimal alignment of nucleotide sequences or amino acid sequences to be compared, addition or deletion (e.g. gap and the like) may be permitted. The sequence identity can be calculated by performing homology analysis to produce alignment using a program such as FASTA[Pearson & Lipman, Proc. Natl. Acad. Sci. USA,4, 2444-2448(1988) ], BLAST[Altschul et al., Journal of Molecular Biology, 215, 403-410(1990) 1. CLUSTAL W[Thompson. Higgins&Gibson, Nucleic Acid Research, 22, 4673-4680(1994a) ]and the like. The program is generally available at the website (http://www.ddbj.nig.ac.jp) of DNA Data Bank of Japan [International DNA Data Bank managed in National Institute of Genetics, Center for Information Biology and DNA Data Bank of Japan ;CIB/DDBJ]. Alternatively, sequence identity can be also obtained using a commercially available sequence analyzing software. Specifically, for example, sequence identity can be calculated by performing homology analysis using GENETYX-WIN Ver. 5 (manufactured by Software Development Co.Ltd.) by a Lipman-Pearson method [Lipman, D. J. and Pearson, W.R., Science, 227, 1435-1441,(1985)] and producing alignment.

Examples of the "stringent condition" described in (f) include condition under which, in hybridization performed according to a conventional method described in Sambrook J., Frisch E. F., Maniatis T., Molecular Cloning 2nd edition, Cold Spring Harbor Laboratory press, for example, a hybrid is formed at 45°C in a solution containing 6xSSC (a solution containing 1.5m NaCl and 0.15 m trisodium citrate is 10xSSC) and, thereafter, this is washed with 2xSSC at 50°C (Molecular Biology, John Wiley & Sons, N. Y. (1989), 6.3.1-6.3.6). A salt concentration in a washing step can be selected from condition from 2xSSC (low stringent condition) to 0. 2xSSC (high stringent condition). A temperature in a washing step can be selected, for example, from condition from room temperature (low stringent condition) to 65°C (high stringent condition). Alternatively, both of a salt concentration and a temperature can be changed.

A protein described in (h) indicates a vesicle-fusing ATPase presents in a cotton aphid among an insect vesicle-fusing ATPase, and includes a protein comprising an amino acid sequence described in (a).

In addition, a protein of the group A includes a protein comprising an amino acid sequence of an insect vesicle-fusing ATPase described in (g) and, more preferably includes, a protein in which when aligned with the amino acid sequence of SEQ ID NO: 1 so that maximum sequence identity is obtained, amino acid residues at positions corresponding to (I) position 314, (II) position 341, (III) position 343, (IV) position 421, (V) position 529, (VI) position 547, (VII) position 584, (VIII) position 587, (IX) position 613 and (X) position 623 of the amino acid sequence of SEQ ID NO: 1 are (I) glutamic acid (at position corresponding to 314), (II) valine (at position corresponding to 341), (III) asparagine (at position corresponding to 343), (IV) isoleucine (at position corresponding to 421). (V) alanine (at position corresponding to 529), (VI) asparagine (at position corresponding to 547), (VII) leucine (at position corresponding to 584), (VIII) arginine (at position corresponding to 587), (IX) glycine (at position corresponding to 613) and (X) threonine (at position corresponding to 623), respectively. Herein, the "aligned with the amino acid sequence of SEQ ID NO: 1 so that maximum sequence identity is obtained" means that sequence identity of a plurality of amino acid sequences to be analyzed including the amino acid sequence of SEQ ID NO: 1 is analyzed by a program such as FASTA, BLAST, CLUSTAL W described above, and they are aligned. By aligning a plurality of sequences by the method, positions of conserved amino acid residues in each amino acid sequence can be determined regardless of insertion or deletion in an amino acid sequence. It is thought that conserved amino acid residues are present at the same position in the three-dimensional structure of the proteins of interest, and it is presumed that similar effect is possessed regarding specific function of the proteins of interest. For example, when insect vesicle-fusing ATPase including the vesicle-fusing ATPase sequence of which is disclosed in the present invention, are aligned with the amino acid sequence of SEQ ID NO: 1 so that maximum sequence identity of amino acid sequences is obtained, it is shown that amino acid residues at positions corresponding to (I) position 314, (II) position 341, (III) position 343, (IV) position 421, (V) position 529, (VI) position 547, (VII) position 584, (VIII) position 587, (IX) position 613 and (X) position 623 of the amino acid sequence of SEQ ID NO: 1 are (I) glutamic acid (at position corresponding to 314), (II) valine (at position corresponding to 341), (III) asparagine (at position corresponding to 343), (IV) isoleucine (at position corresponding to 421), (V) alanine (at position corresponding to 529), (VI) asparagine (at position corresponding to 547), (VII) leucine (at position corresponding to 584), (VIII) arginine (at position corresponding to 587), (IX) glycine (at position corresponding to 613) and (X) threonine (at position corresponding to 623), respectively.

A substance having a pesticidal ability can be screened by using a method of assaying a pesticidal ability by measuring a pesticidal ability or controlling effect on the aforementioned pests.

Alternatively, a substance having a pesticidal ability can be also screened by the method of assaying a pesticidal ability using a vesicle-fusing ATPase. Specifically, when it has been identified that a pesticidal ability of a test substance is a certain value or more, or a certain value or less using the method of assaying a pesticidal ability using a vesicle-fusing ATPase, a substance having a pesticidal ability can be screened by selecting the substance.

Since a substance selected by the screening method has a pesticidal ability, it can be used as a pesticidal agent containing the substance or an agriculturally acceptable salt as an active ingredient.

Control of pests can be usually performed by application an effective amount of a pesticidal agent to a crop protected, a pest, or a habitat of a pest.

When a pesticidal agent is used for agriculture and forestry, its application amount is usually 0.1 to 1000g in terms of an amount of a pesticidal agent per 1000 m$^2$. When a pesticidal agent is formulated into an emulsion, a water-dispersible powder, a flowable preparation, a microcapsule preparation or the like, the agent is usually applied by diluting with water to an active ingredient concentration of 1 to 10,000 ppm, and spraying this and, when a pesticidal agent is formulated into a granule, a powder or the likes, the agent is usually applied as it is.

A pesticidal agent can be used by foliage-treating a plant such as a crop and the like which should be protected from pests, and can be also used by treating a seedbed before a plantlet of a crop is transplanted, or a planting hole or a strain base at planting. Further, for the purpose of controlling pests habiting a soil of a cultivating land, the agent may be used by treating the soil. Alternatively, the agent may be used by a method of winding a resin preparation which has been processed to a sheet or a string, on a crop, stretching the preparation near a crop and/or spreading on a soil surface of a strain base.

When a pesticidal agent is used as a pest controlling agent for preventing an epidemic, an emulsion, awater-dispersible powder, a flowable or the like is usually applied by diluting with water so that an active ingredient concentration becomes 0.01 to 10,000 ppm, and an oily agent, an aerosol, a fumigant, a poison bait or the like is applied as it is.

Examples of one utility of a pesticidal agent include control of an external parasite of a livestock such as cattle, sheep, goat, and chicken, or a small animal such as dog, cat, rat, and mouse, in this case, the agent can be administered to an animal by the veterinarily known method. As a specific administration method, when systemic control is intended, the agent is administered, for example, by a tablet, mixing in feed, suppository, injection (intramuscular, subcutaneous, intravenous, intraperitoneal etc.) and the like, when non-systemic control is intended, the agent is used by a method of spraying an oily agent or an aqueous liquid agent, performing pour on or spot on treatment, washing an animal with a shampoo preparation or attaching a resin preparation which has been processed into a necklace or a ear tag to an animal. An amount of a pesticidal agent when administered to an animal body is usually in a range of 0.1 to 1,000 mg as expressed by total amount of a compound A and a compound B per 1kg of an animal.

An application amount and an application concentration of them are both different depending on the situations such as a kind of a preparation, an application time, an application place, an application method, a kind of a pest, a damage degree and the like, can be increased or decreased regardless of the aforementioned range, and can be appropriately selected.

The aforementioned pesticidal agent can be used in the method of controlling pests as described above.

In addition, on the other hand, a pest can be also controlled by identifying a substance having a pesticidal ability evaluated by the aforementioned method of assaying a pesticidal ability possessed by a pest substance, having a first step and a second step using a vesicle-fusing ATPase selected from group A, and contacting the identified substance having a pesticidal ability with a pest. Herein, as a method of contacting an identified substance having a pesticidal ability with a pest, the aforementioned preparation method, application method and the like can be used.

An amino acid sequence shown in the group B is an amino acid sequence of insect vesicle-fusing ATPase comprising any amino acid sequence of the following (a) to (g).

> (a) the amino acid sequence of SEQ ID NO: 1;
> (b) an amino acid sequence with deletion, addition or substitution of one or more amino acids in the amino acid sequence of SEQ ID NO: 1, wherein said amino acid sequence has vesicle-fusing ATPase activity;
> (c) an amino acid sequence that has sequence identity of 75% or more to the amino acid sequence of SEQ ID NO: 1, wherein said amino acid sequence has vesicle-fusing ATPase activity;
> (d) the amino acid sequence encoded by the nucleotide sequence of SEQ ID NO: 2;
> (e) an amino acid sequence encoded by a nucleotide sequence that has sequence identity of 75 % or more to the nucleotide sequence of SEQ ID NO: 2, wherein said amino acid sequence has vesicle-fusing ATPase activity;
> (f) an amino acid sequence encoded by a polynucleotide wherein said polynucleotide hybridizes under a stringent condition to a polynucleotide comprising a nucleotide sequence complementary to the nucleotide sequence of SEQ ID NO: 2, wherein said amino acid sequence has vesicle-fusing ATPase activity; and
> (g) an amino acid sequence of a cotton aphid vesicle-fusing ATPase.

Among amino acid sequences of the group B, a difference which can be recognized between an amino acid sequence represented by (a) and amino acid sequences represented by (b), (c), (e), (f) and (g) is deletion, substitution, addition or the like of a part of amino acids. These include, for example, deletion due to processing which the protein having an amino acid sequence represented by (a) undergoes in a cell. In addition, examples include deletion, substitution, addition and the like of an amino acid generated by naturally occurring gene mutation due to a spices difference or an individual difference of an organism from which the protein is derived, or gene mutation which is artificially introduced by a site-directed mutagenesis, a random mutagenesis, mutation treatment or the like.

The number of amino acids undergoing the deletion, substitution, addition or the like may be the number in a range that the peptidase activity of a vesicle-fusing ATPase can be found out. In addition, examples of substitution of an amino acid include substitution with an amino acid which is similar in characteristic in hydrophobicity, charge, pH and steric structure. Specific examples of the substitution include substitution in an group of (1) glycine, alanine; (2) valine, isoleucine, leucine; (3) aspartic acid, glutamic acid, asparagine, glutamine, (4) serine, threonine; (5) lysine, arginine; (6) phenylalanine, tyrosine and the like.

Examples of a procedure of artificially introducing the deletion, addition or substitution of an amino acid (hereinafter, collectively referred to as alteration of amino acid in some cases) include a procedure of introducing site-directed mutation into a DNA encoding an amino acid sequence represented by (a) and, thereafter, expressing this DNA by a conventional method. Herein, examples of a site-directed mutagenesis include a method utilizing amber mutation (gapped duplex method, Nucleic Acids Res., 12, 9441-9456(1984)), a method by PCR using primers for mutation introduction, and the like. In addition, examples of a procedure of artificially altering an amino acid include a procedure of randomly introducing mutation into a DNA encoding an amino acid sequence represented by (a) and, thereafter, expressing this DNA by a conventional method. Herein, examples of a method of randomly introducing mutation include a method of performing PCR using a DNA encoding any of the aforementioned amino acid sequences as a template, and using a primer pair which can amplify each full length DNA at reaction condition under which an addition amount of each of dATP, dTTP, dGTP and dCTP used as a substrate is changed from a conventional concentration, or at reaction condition under which a concentration of $Mg^{2+}$ promoting a polymerase reaction is increased from a conventional concentration. Examples of the procedure of PCR include a method described, for example, in Method in Molecular Biology, (31), 1994, 97-112. Another example includes a method described in WO 0009682.

Herein, the "sequence identity" refers to identity between two nucleotide sequences or two amino acids. The "sequence identity" is determined by comparing two sequences which are aligned in the optimal state over an all region of sequences to be compared. Herein, in optimal alignment of nucleotide sequences or amino acid sequences to be compared, addition or deletion (e.g. gap and the like) may be permitted. The sequence identity can be calculated by performing homology analysis to produce alignment using a program such as FASTA [Pearson & Lipman, Proc. Natl. Acad. Sci. USA,4, 2444-2448(1988) ], BLAST [Altschul et al., Journal of Molecular Biology, 215, 403-410(1990)], CLUSTAL W [Thompson, Higgins&Gibson, Nucleic Acid Research, 22, 4673-4680(1994a)] and the like. The program is generally available at the website (http://www.ddbj.nig.ac.jp) of DNA Data Bank of Japan [International DNA Data Bank managed in National Institute of Genetics, Center for Information Biology and DNA Data Bank of Japan ;CIB/DDBJ]. Alternatively, sequence identity can be also obtained using a commercially available sequence analyzing software. Specifically, for example, sequence identity can be calculated by performing homology analysis using GENETYX-WIN Ver. 5 (manufactured by Software Development Co.Ltd.) by a Lipman-Pearson method [Lipman, D. J. and Pearson, W.R., Science, 227, 1435-1441,(1985)] and producing alignment.

Examples of the "stringent condition" described in (f) include condition under which, in hybridization performed according to a conventional method described in Sambrook J., Frisch E. F., Maniatis T., Molecular Cloning 2nd edition. Cold Spring Harbor Laboratory press, for example, a hybrid is formed at 45°C in a solution containing 6xSSC (a solution containing 1.5 m NaCl and 0.15 m trisodium citrate is 10xSSC) and, thereafter, this is washed with 2xSSC at 50°C (Molecular Biology, John Wiley & Sons, N. Y. (1989), 6.3.1-6.3.6). A salt concentration in a washing step can be selected from condition from 2xSSC (low stringent condition) to 0.2xSSC (high stringent condition). A temperature in a washing step can be selected, for example, from condition from room temperature (low stringent condition) to 65°C (high stringent condition). Alternatively, both of a salt concentration and a temperature can be changed.

A protein having an amino acid sequence described in (g) indicates a vesicle-fusing ATPase presents in a cotton aphid among an insect vesicle-fusing ATPase, and includes a protein comprising an amino acid sequence described in (a).

A protein having an amino acid sequence shown in the group B can be prepared, for example, according to a method described later using a polynucleotide encoding an amino acid sequence shown in the group B.

An insect vesicle-fusing ATPase can be used as a reagent that provides an indicator to evaluate a pesticidal activity. Specifically, for example, an insect vesicle-fusing ATPase can be used as a reagent that provides an indicator to evaluate a pesticidal activity by using as a vesicle-fusing ATPase used in the method of assaying a pesticidal ability using a vesicle-fusing ATPase. In addition, a more specific method can be performed according to the aforementioned method of measuring the activity of a vesicle-fusing ATPase.

In addition, when an insect vesicle-fusing ATPase is used as a reagent that provides an indicator to evaluate a pesticidal activity, more preferably, it is desirable that an insect vesicle-fusing ATPase is a vesicle-fusing ATPase having an amino acid sequence shown in the group B.

A polynucleotide having a nucleotide sequence encoding an amino acid sequence shown in the group B (hereinafter, referred to as polynucleotide group B in some cases) has a nucleotide sequence from which a protein having an amino acid sequence can be produced shown in the group B, in a cell of an organism or an in vitro translation system. A polynucleotide group B may be a DNA cloned from a nature, a DNA in which deletion, substitution or addition of a nucleotide is introduced into a DNA cloned from a nature, for example, by a site-directed mutagenesis or a random mutagenesis, or an artificially synthesized DNA. Specifically, examples include a polynucleotide comprising a nucleotide sequence represented by SEQ ID NO: 2.

<First obtaining method>

For example, a method of obtaining a polynucleotide comprising the nucleotide sequence of SEQ ID NO: 2 included in the polynucleotide group B will be shown below. As a step, total RNA is obtained from cotton aphids, cDNA library is synthesized, and PCR amplification is performed, thereby, a polynucleotide of interest can be obtained.

A population of adults and larvae of *Aphis gossypii,* which have been reared on leaves of potted cucumber, is scraped from the surface of the leaves with a small brush, and 630 mg of the obtained population is crushed into a powder in liquid nitrogen using a mortar and a pestle. From the resulting frozen crushed powder, RNA is isolated using a RNA extracting reagent ISOGEN (manufactured by Nippon Gene) as follows. After 10 ml of ISOGEN is added to the frozen crushed powder in the mortar, the crushed powder is ground for 10 minutes while kept on ice. After grinding, a fluid sample is transferred to a 15 ml tube with a pipette, and 2ml of chloroform (manufactured by Wako Pure Chemical Industries, Ltd.) is added thereto. Immediately, the mixture is vigorously shaken for 15 seconds and then left at rest at room temperature for 3 minutes. Then, the resulting mixture is centrifuged at 12,000xg at 4°C for 15 minutes, and each 5 ml of aqueous layer are transferred to two new tubes. After 5 ml of ISOGEN is added to each tube, the mixture was immediately shaken vigorously for 15 seconds, and left at rest at room temperature for 3 minutes. Then, the resulting mixture is centrifuged at 12,000xg at 4°C for 15 minutes, and each 10 ml of aqueous layer are transferred to new 50 ml tubes, respectively. Subsequently, 10 ml of isopropanol (manufactured by Wako Pure Chemical Industries, Ltd.) is added to each tube, and the mixture is kept on ice for 30 minutes . The resulting mixture is centrifuged at 12,000xg at 4°C for 10 minutes to precipitate RNA. After the supernatant is removed, 20 ml of 70% ethanol is added to the residue. The resulting mixture is centrifuged at 10,000xg at 4°C for 5 minutes. After the supernatant is removed, the precipitate of total RNA is slightly dried and then dissolved in 1 ml of commercially available RNase-free water (Nacalai Tesque, Inc.). An absorbance of the prepared total RNA is measured at 260 nm to calculate a concentration according to a conventional method.

RT-PCR is performed employing total RNA of cotton aphid obtained by the aforementioned method as a template, and using random primers (manufactured by Invitrogen) and superscript III (manufactured by Invitrogen) according to the manual annexed to the reagent, to synthesized a first-strand cDNA.

PCR is performed employing cDNA library of cotton aphid obtained by the aforementioned method as a template, and using an oligonucleotide primer comprising the nucleotide sequence of SEQ ID NO: 3 and an oligonucleotide primer comprising the nucleotide sequence of SEQ ID NO: 4 as well as a La-Taq polymerase (manufactured by Takara Bio) according to the manual annexed to the reagent. The PCR conditions are those for touchdown PCR as follows: an initial denaturation at 94°C for 10 minutes; followed by 10 cycles of touchdown-PCR, one cycle being 94°C for 20 seconds, 60°C for 20 seconds with a decrease of 1°C per cycle, and 72°C for 2 minutes; followed by 25 cycles of PCR, one cycle being 94°C for 20 seconds, 50°C for 20 seconds, and 72°C for 3 minutes; and followed by 72°C for 7 minutes.

As described above, a polynucleotide comprising the nucleotide sequence of SEQ ID NO: 2 can be obtained.

<Second obtaining method>

Alternatively, a polynucleotide shown in the polynucleotide group B can be also obtained by preparing a polynucleotide with mutation introduced therein by a method utilizing amber mutation which is the aforementioned site-directed mutagenesis, a method by PCR using a primer for introducing mutation or the like, using as a template a polynucleotide comprising the nucleotide sequence of SEQ ID NO: 2.

<Third obtaining method>

Alternatively, a polynucleotide shown in the polynucleotide group B can be also obtained by a hybridization method using a polynucleotide comprising the nucleotide sequence of SEQ ID NO: 2 as a probe. More specifically, the third obtaining method can be performed according to a conventional hybridization described in the aforementioned Sambrook J., Frisch E. F., Maniatis T., Molecular Cloning 2nd edition, published by Cold Spring Harbor Laboratory press.

<Fourth obtaining method>

Alternatively, a polynucleotide shown in the polynucleotide group B can be also obtained by preparing a primer based on an amino acid sequence of the known insect vesicle-fusing ATPase and performing PCR. For isolation of homologues of vesicle-fusing ATPase gene from other insect species such as German cockroach (*Blatella germanica*), degenerate primers are designed using Codehop program (publicly accessible on the website of Blocks Protein Analysis Server operated within the Fred Hutchinson Cancer Research Center at http://blocks.fhcrc.org/blocks/codehop.html), and based on the sequence of the aforementioned cotton aphid-derived vesicle-fusing ATPase gene and the previously-known nucleotide sequences of D. melanogaster (NCBI accession number NM_080138) Manduca sexta (NCBI accession number AF118384), Helicoverpa zea (NCBI accession number AY220909) Anopheles gambiae (NCBI accession number XM_307148), and the like.

Partial sequences of a homologue of vesicle-fusing ATPase gene of a selected insect species are amplified by a series of PCR using first-strand cDNA derived from the insect species as a template. Herein, the first-strand cDNA as a template is prepared by the aforementioned method using Superscript III. Amplification by PCR is performed using a set of degenerate primers as a forward primer and a reverse primer as well as Amplitaq Gold (manufactured by Applied Biosystems) according to the manufacturer's procedure annexed to the reagent. The PCR conditions are those for touchdown PCR as follows: an initial denaturation at 94°C for 10 minutes; followed by 10 cycles of touchdown-PCR, one cycle being 94°C for 30 seconds, 60°C for 1 minute with a decrease of 1°C per cycle, and 72°C for 1 minute and 30 seconds; followed by 25 cycles of PCR, one cycle being 94°C for 30 seconds, 50°C for 1 minute, and 72°C for 1 minute and 30 seconds; and followed by 72°C for 7 minutes. The PCR product is analyzed and purified by agarose gel electrophoresis to obtain DNA of interest. Further, the obtained DNA is cloned into the pCR4-TOPO vector (manufactured by Invitrogen), and sequenced.

Then, primers specific for the resulting partial sequences of the insect homologue of vesicle-fusing ATPase gene are designed, and 3'RACE PCR or 5'RACE PCR is performed in order to obtain a full-length sequence of the gene. 3' and 5'RACE PCRs are performed employing first-strand cDNA prepared from the insect total RNA as a template and using SMART PCR cDNA Synthesis Kit (manufactured by Clontech) or 5'/3' RACE Kit, 2nd Generation (manufactured by Roche) according to the manufacturer's instructions annexed to the kit.

When using SMART PCR cDNA Synthesis Kit, In 3'RACE and 5'RACE reactions, universal primer mix (UPM) contained in SMART PCR cDNA Synthesis Kit is used in combination with a forward primer or a reverse primer which is specific for the sequence of interest. The PCR conditions are those for touchdown PCR as follows: an initial denaturation at 94°C for 10 minutes; followed by 10 cycles of touchdown-PCR, one cycle being 94°C for 30 seconds, 60°C for 1 minute with a decrease of 1°C per cycle, and 72°C for 2 minutes; followed by 25 cycles of PCR, one cycle being 94°C for 30 seconds, 50°C for 1 minute, and 72°C for 2 minutes; and followed by 72°C for 7 minutes. The resulting PCR product is analyzed and purified by agarose gel electrophoresis to obtain DNA of interest. Further, the obtained DNA is cloned into the pCR4-TOPO vector (manufactured by Invitrogen), and sequenced.

When a distinct amplification product is not obtained by the first-round PCR, nested PCR is performed using the first-round PCR product as a template. As primers, NUP primer contained in SMART PCR cDNA Synthesis Kit is used in combination with a specific forward primer or a specific reverse primer which is designed to bind internal sequence of the first-round PCR product of interest. The PCR conditions are those for touchdown PCR as follows : an initial denaturation at 94°C for 10 minutes; followed by 10 cycles of touchdown-PCR, one cycle being 94°C for 30 seconds, 60°C for 30 seconds with a decrease of 1°C per cycle, and 72°C for 2 minutes; followed by 25 cycles of PCR, one cycle being 94°C for 30 seconds, 50°C for 1 minute, and 72°C for 2 minutes; and followed by 72°C for 7 minutes. The resulting PCR product is analyzed and purified by agarose gel electrophoresis to obtain DNA of interest. Further, the obtained DNA is cloned into the pCR4-TOPO vector (manufactured by Invitrogen), and sequenced.

The above sequencing results reveal 5'-terminal sequence and 3'-terminal sequence, each encoding N-terminal region and C-terminal region of the insect vesicle-fusing ATPase, respectively.

Thus, a polynucleotide shown in the polynucleotide group B can be obtained by PCR by preparing a primer based on an amino acid sequence of the known insect vesicle-fusing ATPase.

A polynucleotide comprising a nucleotide sequence complementary to a polynucleotide sequence of the polynucleotide group B can be used for obtaining a polynucleotide shown in the polynucleotide group B using a hybridization method.

The obtaining method in the present invention comprises a step of detecting a desired polynucleotide by hybridization, a step of identifying the detected desired polynucleotide, and a step of recovering the identified desired polynucleotide. Each step will be explained specifically below.

A step of detecting a desired polynucleotide by hybridization, and a step of identifying the detected desired polynucleotide can be performed by using, as a probe, a polynucleotide having a nucleotide sequence having complementarity to a nucleotide sequence of a polynucleotide group B, according to the method described, for example, in "Molecular Cloning: A Laboratory Manual 2nd edition" (1989), Cold Spring Harbor Laboratory Press, "Current Protocols In Molecular Biology" (1987), John Wiley & Sons,Inc.ISBNO-471-50338-X and the like.

Specifically, for example, a DNA comprising a nucleotide sequence complementary to the nucleotide sequence of SEQ ID NO: 2 is labeled with a radioisotope or a fluorescently labeled by the known method using Random Primed DNA Labelling Kit (manufactured by Boehringer), Random Primer DNA Labelling Kit Ver.2 (manufactured by TAKARA SHUZO Co., Ltd.), ECL Direct Nucleic Acid Labelling and Ditection System (manufactured by Amersham Biosciences), or Megaprime DNA-labelling system (manufactured by Amersham Biosciences),and this can be used as probe.

Examples of condition for hybridization include stringent condition, and specifically, examples include condition under which incubation is performed at 65°C in the presence of 6xSSC (0.9M NaCl, 0.09M sodium citrate), a 5xDenhart's solution (0.1%(w/v) Ficoll 400, 0.1%(w/v) polyvinylpyrrolidone, 0.1% BSA), 0.5%(w/v) SDS and 100 μg/ml denatured

salmon spermatozoon DNA, or in a DIG EASY Hby solution (Boehringer Mamnnheim) containing 100 μg/ml denatured salmon spermatozoon DNA, then, incubation is performed two times at room temperature for 15 minutes in the presence of 1xSSC (0.15 m NaCl, 0.015 m sodium citrate) and 0.5%SDS and, further, incubation is performed at 68°C for 30 minutes in the presence of 0.1xSSC (0.015 m NaCl. 0.0015 m sodium citrate) and 0.5%SDS.

More specifically, for example, a probe labeled with $^{32}$P can be made by employing a polynucleotide comprising a nucleotide sequence complementary to a nucleotide sequence of a polynucleotide group B as a template, using Megaprime DNA-labelling system (manufactured by (Amersham Pharmacia Biotech) and using a reaction solution designated in a kit. Colony hybridization is performed using this probe according to a conventional method, incubation is performed at 65°C in the presence of 6xSSC (0. 9M NaCl, 0 .09M sodium citrate), a 5xDerihart's solution (0.1%(w/v) Ficoll 400, 0.1%(w/v) polyvinylpyrrolidone, 0.1%BSA), 0.5%(w/v) SDS and 100 μg/ml denatured salmon spermatozoon DNA, or in a DIG EASY Hyb solution (Boehringer Mannheim) containing 100 μg/ml denatured salmon spermatozoon DNA, then, incubation is performed two times at room temperature for 15 minutes in the presence of 1xSSC (0.15 m NaCl, 0.015 m sodium citrate) and 0.5%SDS and, further, incubation is performed at 68°C for 30 minutes in the presence of 0.1xSSC (0.015 m NaCl, 0.0015 m sodium citrate) and 0.5%SDS, thereby, (a colony containing) a hybridizing polynucleotide can be detected. Thus, a desired polynucleotide can be detected by hybridization, and the detected desired polynucleotide can be identified.

For recovering the identified desired polynucleotide, a plasmid DNA can be recovered from a colony containing the polynucleotide detected and identified by the aforementioned method, for example, according to a method such as the alkali method described in "Molecular Cloning: A Laboratory Manual 2nd edition" (1989), Cold Spring Harbor Laboratory Press. A nucleotide sequence of the recovered desired polynucleotide (plasmid DNA) can be confirmed by a Madam Gilbert method (described, for example, in Maxam,A.M& W.Gilbert, Proc.Natl.Acad.Sci.USA, 74, 560, 1977 etc. or a Sanger method (described, for example, in Sanger,F. & A.R.Coulson, J.Mol.Biol., 94, 441, 1975.Sanger,F, & Nicklen and A.R.Coulson., Proc.Natl.Acad.Sci.USA, 74, 5463, 1977 etc.). Thereupon, for example, commercially available Termo Seqenase II dye terminator cycle sequencing kit (manufactured by Amersham biosciences), Dye Terminator Cycle Sequencing FS Ready Reaction Kit (manufactured by Applied Biosystems) and the like can be used.

A polynucleotide comprising a partial nucleotide sequence of a nucleotide sequence of the polynucleotide group B or a nucleotide sequence complementary to the partial nucleotide sequence can be used for obtaining a polynucleotide shown in the polynucleotide group B using PCR. More specifically, examples include a polynucleotide comprising a nucleotide sequence of SEQ ID NO: 3 or 4. The obtaining method in the present invention includes a step of amplifying a desired polynucleotide by PCR, a step of identifying the amplified desired polynucleotide, and a step of recovering the identified desired polynucleotide. Each step will be specifically explained below.

In a step of amplifying a desired polynucleotide by PCR, specifically, a DNA designed and synthesized from a partial nucleotide sequence of a nucleotide sequence of a polynucleotide group B or a nucleotide sequence complementary to the partial nucleotide sequence, based on an about 20bp to about 40bp nucleotide sequence, for example, a nucleotide sequence selected from a nucleotide sequence of SEQ ID NO: 2 and a sequence complementary to the nucleotide sequence of SEQ ID NO: 2 can be used as a primer set. Examples of a primer set include a set of a polynucleotide comprising a nucleotide sequence represented by SEQ ID NO: 3 and a polynucleotide comprising the nucleotide sequence of SEQ ID NO: 4. A PCR reaction solution is prepared, for example, by adding a reaction solution designated by a commercially available PCR kit to a cDNA library prepared by the aforementioned method. Reaction condition can be changed depending on a primer set to be used, and for example, condition under which after incubation at 94°C for 10 seconds, around 40 cycles is repeated, 1 cycle being 94°C for 15 seconds, 60°C for 15 seconds, and 72°C for 3 minutes and, further, incubation is performed at 72°C for 3 minutes, condition under which incubation is performed at 94°C for 2 minutes, thereafter, incubation is performed at about 8°C for 3 minutes and, thereafter, around 40 cycles is repeated, 1 cycle being 94°C for 30 seconds, 55°C for 30 seconds, and 72°C for 4 minutes, or condition under which 5 to 10 cycles is performed, 1 cycle being incubation at 94°C for 5 seconds and, then, 72°C for 4 minutes and, further, around 20 to 40 cycles is performed, 1 cycle being incubation at 94°C for 5 seconds and, then, 70°C for 4 minutes, can be used. In the PCR, for example, PfuUltra High Fidelity polymerase (manufactured by Stratagene), Amplitaq Gold (manufactured by Applied Biosystems), Takara Heraculase (Trademark) (manufactured by TAKARA SHUZO Co., Ltd.), a DNA polymerase contained in Advantage cDNA PCR Kit (manufactured by Clonetech) , TaKaRa Ex Taq (manufactured by TAKARA SHUZO Co., Ltd.), PLATINUMTM PCR SUPER Mix (manufactured by Lifetech Oriental) can be used.

Identification of a desired polynucleotide amplified by PCR can be performed by measuring a molecular weight by agarose gel electrophoresis according to the method described in "Molecular Cloning: A Laboratory Manual 2nd edition" (1989), Cold Spring Harbor Laboratory Press. In addition, regarding the amplified desired polynucleotides, a sequencing reaction is performed using a commercially available DNA sequencing reaction kit, for example, Dye Terminator Cycle Sequencing FS Ready Reaction Kit (manufactured by Applied Biosystems) according to a manual annexed to the kit, and the nucleotide is analyzed using a DNA sequencer 3100 (manufactured by Applied Biosystems), thereby, a nucleotide sequence of the amplification fragment can be read.

Examples of a method of recovering the identified desired polynucleotide include a method of purifying and recovering the aforementioned polynucleotide identified by agarose gel electrophoresis from an agarose gel according to the method described in "Molecular Cloning: A Laboratory Manual 2nd edition" (1989), Cold Spring Harbor Laboratory Press . In addition, the thus recovered polynucleotide or a desired polynucleotide amplified by PCR can be cloned into a vector according to a conventional method described in "Molecular Cloning: A Laboratory Manual 2nd edition" (1989), Cold Spring Harbor Laboratory Press , and "Current Protocols In Molecular Biology" (1987), John Wiley & Sons,Inc. ISBN0-471-50338-X. Examples of a vector to be used include pUCA119 (manufactured by TAKARA SHUZO Co., Ltd.), pTVA118N (manufactured by TAKARA SHUZO Co., Ltd.), pBluescriptII (manufactured by Toyobo Co., Ltd.), pCR2.1-TOPO (manufactured by Invitrogen) and the like. In addition, a nucleotide sequence of the cloned polynucleotide can be confirmed by a Maxam Gilbert method (described, for example, in Maxam,A.M & W.Gilbert, Proc.Natl.Acad.Sci.USA, 74, 560, 1977 or a Sanger method (described, for example, in Sanger,F. & A.R.Coulson, J.Mol,Biol., 94, 441, 1975, Sanger,F, & Nicklen and A.R.Coulson., Proc.Natl.Acad.Sci.USA, 74, 5463, 1977). Thereupon, for example, a commercially available Thermo Seqenase II dye terminator cycle sequencing kit (manufactured by Amersham biosciences), Dye Terminator Cycle Sequencing FS Ready Reaction Kit (manufactured by Applied Biosystems) and the like can be used.

In addition, a polynucleotide having a partial nucleotide sequence of a nucleotide sequence of the polynucleotide group B or a nucleotide sequence complementary to the partial nucleotide sequence can be used for obtaining a polynucleotide shown in the polynucleotide group B using not only a PCR method, but also the aforementioned hybridization method. More specifically, examples include a polynucleotide comprising a nucleotide sequence represented by SEQ ID NO: 3 or 4.

Examples of a method for preparing a protein comprising an amino acid sequence shown in the group B include a method of culturing a transformant with a polynucleotide selected from a polynucleotide group B introduced therein, and recovering the produced protein. In addition, for preparing a transformant used herein, it is a work such as preparation of a circular polynucleotide containing a polynucleotide in which a polynucleotide selected from a polynucleotide group B is operably ligated to a bacteriophage promoter. The method will be explained in detail below.

In addition a vesicle-fusing ATPase shown in a group A which is used in the method of assaying a pesticidal activity using a vesicle-fusing ATPase can be prepared and obtained by the similar method, using a polynucleotide comprising a nucleotide sequence encoding a vesicle-fusing ATPase used.

A bacteriophage promoter means a promoter of a gene contained in a bacteriophage genome. Among them, examples of a promoter of bacteriophage used for expressing a foreign gene include a promoter of T7 RNA polymerase gene, T3 RNA polymerase gene and SP6 RNA polymerase gene.

In the present invention, "operably linked" means that a polynucleotide containing a gene of interest is linked downstream of a polynucleotide containing a promoter sequence so that the gene of interest can be transcribed in a used transcription system. Specifically, for example, when a promoter of T7 RNA polymerase gene described later is used, a polynucleotide containing a gene of interest may be linked downstream of a promoter of T7 RNA polymerase gene. In addition, for example, when a promoter other than T7 RNA polymerase gene promoter is used, it is also possible to link a polynucleotide containing a gene of interest downstream of a polynucleotide containing a promoter sequence other than T7 RNA polymerase gene promoter. More specifically, for example, when a plasmid pET41b(+)(Novagen) vector utilizing T7 RNA polymerase gene promoter is used, the polynucleotide can be operably linked by ligating a gene of interest into a restriction enzyme site such as NcoI , EcoRV , BamHI , EcoRI , StuI, PstI, SacI, SalI, HindIII, Not, EagI and XhoI located downstream of T7 RNA polymerase gene promoter.

In the present invention, the "circular polynucleotide" is a polynucleotide which has been made to be circular by binding of ends of the polynucleotide strand, and examples include chromosomal DNAs of many bacteria in addition to a plasmid DNA, a bacmid DNA and the like.

A plasmid DNA is a relatively low-molecular circular polynucleotide, and examples include pET (manufactured by Takara Mirus Bio Inc.) and pBluescriptII (manufactured by Stratagene), used for cloning and expression in E. coli. Additional examples include pFastBacl, pFastBac HT A, pFastBac HT B, pFastBac HT C, pFastBac Dual, pBlueBacII (manufactured by Invitrogen), pAcSG2 (manufactured by Pharmingen) and the like, which contain a baculovirus expression cassette.

The bacmid is a high molecular weight DNA that consists of a BAC (bacterial artificial chromosome) that contains the entire baculoviral genome, for example bMON14272 (136 kb) that is present in DH10Bac™ E. *coli* cells (invitrogen). Bacmid DNA propagates as a large plasmid in *E. coli* cells and may contain an expression cassette for expression of a foreign gene under control of a baculoviral promoter.

A circular polynucleotide in which a polynucleotide comprising a nucleotide sequence encoding an amino acid sequence shown in the group B is operably linked to a bacteriophage promoter is specifically, for example, a circular polynucleotide containing a DNA comprising a cotton aphid vesicle-fusing ATPase gene operably linked to a bacteriophage T7 RNA polymerase promoter, and can be prepared and obtained, for example, according to the following methods.

DNA fragment containing the vesicle-fusing ATPase gene is amplified by PCR, using a plasmid DNA containing a cotton aphid vesicle-fusing ATPase gene cloned in accordance with the aforementioned method as a template, with a primer specific to the vesicle-fusing ATPase gene and a primer specific to the vesicle-fusing ATPase gene to which a XhoI restriction site is added. The resulting PCR products are cleaved with XhoI, and the obtained approximately 1.4 kbp of DNA fragment containing the cotton aphis vesicle-fusing ATPase gene is ligated to a Plasmid vector pET41b(+) (manufactured by Novagen) digested with EcoRV and XhoI in advance. The plasmid obtained in this way is one example of circular polynucleotide containing DNA fragment comprising the cotton aphis vesicle-fusing ATPase gene operably linked to bacteriophage T7 RNA polymerase promoter.

Similarly, a circular polynucleotide can be prepared by ligating nucleotides encoding an amino acid sequence shown in the group B to a vector.

In the present invention, the "origin of replication" is the specific DNA sequence necessary for replicating itself in a host cell. Examples of origin of replication include colE1 and f1 for bacterial plasmids.

A transformant is a eukaryotic cell or a prokaryotic cell which has been genetically altered by introduction of a foreign polynucleotide into a cell. Examples of a transformant include an Escherichia coli cell transformed by introduction of a plasmid used for gene cloning or gene expression in E. coli, such as pET (Novagen) or pBluescript II (Stratagene). In addition, examples of the technique of introducing a DNA into a host cell include transformation, transfection, protoplast fusion, lipofection, electroporation and the like.

Examples of a transformant in which a polynucleotide encoding an amino acid sequence shown in the group B is introduced include transformed Escherichia coli in which a DNA comprising a cotton aphid vesicle-fusing ATPase gene operably linked to a bacteriophage T7 RNA polymerase promoter is introduced. Specifically, the transformant can be prepared according to the following method.

A transformant can be prepared by introducing into an Escherichia coli cell a plasmid vector pET41b(+) (Novagen) in which a DNA containing a cotton aphid vesicle-fusing ATPase gene is inserted between EcoRV site and Xho I site, according to the method described in "Molecular Cloning: A Laboratory Manual 2nd edition" (1989), Cold Spring Harbor Laboratory Press. Alternatively, a transformant can be also prepared by transforming E. coli using the aforementioned plasmid DNA into which a fragment containing a bacteriophage T7 RNA polymerase promoter and a cotton aphid vesicle-fusing ATPase gene is inserted, according to a method described in a manual annexed to Escherichia coli BL21 (DE3) competent cells (Invitrogen).

A vesicle-fusing ATPase can be prepared by culturing a transformant prepared by the aforementioned method, and recovering the produced insect-derived vesicle-fusing ATPase.

Vesicle-fusing ATPase protein may be produced by a recombinant *E. coli* expression system. This system is the most frequently used prokaryotic expression system for the high-level production of heterologous proteins. *E. coli* is genetically and physiological the best characterized organism known, it is easy to manipulate, many tools are available, it is able to grow very fast, it grows on cheap complex or well-defined minimal media and it has an extremely high capacity to synthetize heterologous protein.

Alternatively, vesicle-fusing ATPase protein may be produced by for example a recombinant baculovirus/Sf9 cell expression system. This system is one of the most powerful and versatile eukaryotic expression systems available, and may be used to express heterologous genes from many different sources, including fungi, plants, bacteria and viruses.

In addition, an insect-derived vesicle-fusing ATPase produced by culturing a transformant is lysed by a method such as sonication, French press, and Dyno mill, and recovered in a form contained in a cell crude extract, and a purified protein can be obtained by using a procedure conventionally used in enzyme purification such as ion exchange column chromatography, reverse phase column chromatography, gel filtration column chromatography and the like. Alternatively, when it is devised that an insect-derived vesicle-fusing ATPase is produced in a form with His-tag, a purified protein can be obtained rapidly from a cell crude extract by affinity column chromatography which specifically recognizes and binds to the His-tag. By the method, an insect-derived vesicle-fusing ATPase can be prepared.

Alternatively, vesicle-fusing ATPase protein may be produced by for example a recombinant baculovirus/Sf9 cell expression system. For example, an insect-derived vesicle-fusing ATPase can be prepared by culturing a transformed insect cell with a DNA fragment containing a cotton aphid vesicle-fusing ATPase gene operably linked to a polyhedron promoter of baculovirus, and grinding the cell with a French press, followed by purification with column chromatography.

To be more concrete, for example, a recombinant E. coli containing the DNA fragment comprising the cotton aphid vesicle-fusing ATPase gene operably linked to the bacteriophage T7 RNA polymerase promoter, which prepared by the above method, is rotary cultured overnight at 37°C, 250 rpm. In the next morning, the culture is diluted 1/25 in a new culture medium, and grown at 37°C, 250rpm. When the culture reaches an OD of 0.8 at 600 nm, IPTG is added to the culture until a final concentration of 10$\mu$M. After incubating at 25°C, 60 rpm for 4 days, the culture is centrifuged at 7,000 rum for 10 minutes to collect the E. coli. To collected E. coli, breaking buffer (100 mM Hepes/KOH pH 7.5, 500 mM KCl, 5 mM MgCl$_2$ . 2 mM 2-mercaptoethanol, 5 mM ATP, 0. 5 mM, Pefablock, 10 $\mu$g/$\mu$l Leupeptin, 1 $\mu$M Pepstatin A) is added and suspended. Further, the E. coli is lysed with a pressure between 1,300 psi and 1,500 psi, using French press (manufactured by Thermo Spectronic), in accordance with the method described in the attached instruction. The French pressed solution is centrifuged at 14,000 rpm, 2°C, for 60 minutes, and the resulting supernatant is filtrated through a 0.45 μm filter. The sample is injected into HiTrap Chelating HP (manufactured by Amersham biosciences) column or HisTrap HP (manufactured by Amersham biosciences) column, which is equilibrated with His buffer A (20 mM Hepes pH 7.0, 200 mM KCl, 1mM $MgCl_2$, 2mM 2-mercaptoethanol, 0.5 mM ATP, 10% glycerol). Afterwards, the column is washed with a buffer, in which 85% of His buffer A and 15% of His buffer B (20 mM Hepes pH 7.0, 200 mM KCl, 1 mM $MgCl_2$, 2 mM 2 -mercaptoethanol, 0. 5 mM ATP. 500 mM imidazole, 10% glycerol) are mixed, the column is washed by using 5 column volumes of the buffer. Next, the column is washed by using 15 column volumes of buffer, in which 60% of His buffer A and 40% of His buffer B are mixed. Afterwards, 15 column volumes of a buffer in which 30% of His buffer A and 70% of His buffer Bare mixed is prepared and injected into the column. One ml of aliquots of the eluted fraction is pooled and an aliquot is analyzed with SDS-PAGE to determine a fraction including 86 kDa of vesicle-fusing ATPase. These fractions are the solution containing large amount of the target vesicle-fusing ATPase. The solution containing vesicle-fusing ATPase is injected into a column GSTrap FF (manufactured by Amersham biosciences) which is equilibrated with GST buffer A (20 mM Hepes pH 7.0, 200 mM KCl, 1mM $MgCl_2$. 2 mM 2-mercaptoethanol, 10% glycerol). Twenty (20) column volumes of GST buffer is injected to wash the column. Finally, 30 column volumes of the GST buffer B (50 mM Tris-HCl, 10 mM reduced glutathione, 2 mM 2-mercaptoethanol, 10% glycerol) is injected, followed by collection of the eluted fraction. The fraction contains the target vesicle-fusing ATPase dissolved in the GST buffer B. SDS-PAGE analysis of an aliquot of this fraction confirms inclusion of 86 kDa of vesicle-fusing ATPase.

An insect vesicle-fusing ATPase comprising an amino acid sequence shown in the group B can be used as a research tool . For example, it can be used as a research tool for performing study such as assaying of the pestcidal ability, screening of a chemical substance having a pestcidal ability, and the like. In addition, for example, also in study of analyzing action and mechanism of an agent which acts on a vesicle-fusing ATPase, a vesicle-fusing ATPase can be utilized as a research tool.

In addition, polynucleotides encoding amino acid sequences shown in the group B and polynucleotides having a nucleotide sequence having complementarity to them, as well as partial nucleotide,sequences of polynucleotides encoding amino acid sequences shown in the group B, or polynucleotides having nucleotide sequences having complementarity to the partial nucleotide sequences, and a polynucleotide complying a nucleotide sequence represented by SEQ ID NO: 3 or 4 can be used as a research tool. For example, a part of them functions as a polynucleotide used in a method of preparing a vesicle-fusing ATPase as described above. In addition, a part can be used as an important research tool for performing obtaining a polynucleotide shown in a polynucleotide group B using PCR, or obtaining a polynucleotide shown in a polynucleotide group B using hybridization, as described above.

Particularly, upon implementation of screening of a pesticidal agent, they can be used as an experimental tool for an experiment which is performed for screening. Specifically, they can be used as an experimental tool for an experiment which is performed upon implementation of the assaying of a pestcidal ability, screening of a chemical substance having a pestcidal ability, and the like.

Further, the present invention also includes a system which comprises a means to input, store and manage data information of an ability of test substances, wherein said ability is an ability to modulate the activity of an insect vesicle-fusing ATPase (hereinafter, referred to as means a in some cases), a means to query and retrieve the data information based on a desired criterion (hereinafter, referred to as means b in some cases), and a means to display and output the result which is queried and retrieved (hereinafter, referred to as means c in some cases) (hereinafter, referred to as present system in some cases).

First, a means a will be explained. A means a is a means to, after data information of an ability to modulate the activity of an insect-derived vesicle-fusing ATPase possessed by the test substance is inputted, store and manage the inputted information, as described above. The information is inputted by an inputting means 1, and is usually memorized in a memory means 2. Examples of an inputting means include means which can input the information such as a keyboard and a mouse. When inputting and storing managing of the information are completed, a procedure progresses to a next means b. For storing managing the information, a large amount of data may be effectively stored and managed by inputting information having a data structure using a hardware such as a computer, and a software such as OS and database management, and storing the information into a suitable memory device, for example, computer-readable recording medium such as a flexible disc, a photomagnetic disc, CD-ROM, DVD-ROM, and a hard disc.

A means b will be explained. A means b is a means to query and retrieve the data information stored and managed by a means of a based on criterion for obtaining a desired result, as described above. For the information, when criterion for querying and retrieving is inputted by an inputting means 1, and information in conformity with the criterion is selected among the information usually memorized in a memory means 2, a procedure progresses to a next means c. The selected result is usually memorized in a memory means 2 and, further, can be displayed by a displaying outputting means 3.

A means c will be explained. A means c is a means to display and output the result which is queried and retrieved, as described above. Examples of the displaying outputting means 3 include a display, a printer and the like, and the result may be displayed on a display device of a computer, or may be outputted on a paper by printing.

EXAMPLES

The present invention will be explained in more detail below by way of Examples, but the present invention is not limited to these particular Examples.

Example 1 (Extraction of total RNA from cotton aphid and German cockroach)

(1) Extraction of total RNA from cotton aphid.

A population of adults and larvae of cotton aphid (Aphis *gossypii*), which had been reared on leaves of potted cucumber, was scraped from the surface of the leaves with a small brush, and 630 mg of the obtained population was crushed into a powder in liquid nitrogen using a mortar and a pestle. From the resulting frozen crushed powder, RNA was isolated using a RNA extracting reagent ISOGEN (manufactured by Nippon Gene) as follows. After 10 ml of ISOGEN was added to the frozen crushed powder in the mortar, the crushed powder was ground for 10 minutes while kept on ice. After grinding, a fluid sample was transferred to a 15 ml tube with a pipette, and 2ml of chloroform (manufactured by Wako Pure Chemical Industries, Ltd.) was added thereto. Immediately, the mixture was vigorously shaken for 15 seconds and then left at rest at room temperature for 3 minutes. Then, the resulting mixture was centrifuged at 12,000xg at 4°C for 15 minutes, and each 5 ml of aqueous layer were transferred to two new tubes. After 5 ml of ISOGEN was added to each tube, the mixture was immediately shaken vigorously for 15 seconds, and left at rest at room temperature for 3 minutes. Then, the resulting mixture was centrifuged at 12,000xg at 4°C for 15 minutes, and each 10 ml of aqueous layer were transferred to new 50 ml tubes, respectively. Subsequently, 10 ml of isopropanol (manufactured by Wako Pure Chemical Industries, Ltd.) was added to each tube, and the mixture was kept on ice for 30 minutes. The resulting mixture was centrifuged at 12,000xg at 4°C for 10 minutes to precipitate RNA. After the supernatant was removed, 20 ml of 70% ethanol was added to the residue. The resulting mixture was centrifuged at 10,000xg at 4°C for 5 minutes. After the supernatant was removed, the precipitate of total RNA was slightly dried and then dissolve in 1 ml of commercially available RNase-free water (Nacalai Tesque, Inc.). A concentration of the prepared total RNA (calculated from an absorbance at 260 nm) was 6.9mg/ml.

(2:) Extraction of total RNA from German cockroach

Adults, nymphs and oothecae of artificially-reared German cockroach (*Blattella germanica*) were provided as samples. Ten (10) of adult males and 10 of adult females (individuals from each of which ootheca has been removed) were used as an adult sample of 1.1g, 10 of nymph males and 10 of nymph females were used as a nymph sample of 1.0g, and 26 oothecae were used as an ootheca sample of 1.0g. Three kinds of these samples were separately crushed into a powder in liquid nitrogen using separate mortars and pestles. From each of the resulting frozen crushed powders, RNA was isolated using a RNA extracting reagent ISOGEN (manufactured by Nippon Gene) as follows. After 10 ml of ISOGEN was added to the frozen crushed powder in the mortar, the crushed powder was ground for 10 minutes while kept on ice. After grinding, a fluid sample was transferred to a 1.5 ml tube with a pipette, and 2ml of chloroform (manufactured by Wako Pure Chemical Industries, Ltd.) was added thereto. Immediately, the mixture was vigorously shaken for 15 seconds and then left at rest at room temperature for 3 minutes. Then, the resulting mixture was centrifuged at 12,000xg at 4°C for 15 minutes, and each 5 ml of aqueous layer were transferred to two new tubes. After 5 ml of ISOGEN was added to each tube, the mixture was immediately shaken vigorously for 15 seconds, and left at rest at room temperature for 3 minutes. Then, the resulting mixture was centrifuged at 12.000xg at 4°C for 15 minutes, and each 10 ml of aqueous layer were transferred to new 50 ml tubes, respectively. Subsequently, 10 ml of isopropanol (manufactured by Wako Pure Chemical Industries, Ltd.) was added to each tube, and the mixture was kept on ice for 30 minutes. The resulting mixture was centrifuged at 12,000xg at 4°C for 10. minutes to precipitate RNA. After the supernatant was removed, 20 ml of 70% ethanol was added to the residue. The resulting mixture was centrifuged at 10,000xg at 4°C for 5 minutes. After the supernatant was removed, the precipitate of total RNA was slightly dried and then dissolved in 1 ml of commercially available RNase-free water (Nacalai Tesque, Inc.). A concentration of the prepared total RNA (calculated from absorbance at 260 nm) was 1.1 mg/ml in the case of adult-derived total RNA, was 2.5 mg/ml in the case of nymph-derived total RNA, and 1.4 mg/ml in the case of ootheca-derived total RNA.

Example 2 (Isolation of cotton aphid vesicle-fusing ATPase gene)

First-strand cDNA was prepared using total RNA from cotton aphid, random Primers (Invitrogen) and Superscript III (Invitrogen) for RT-PCR according to the manufacturer's procedure of Superscript III.
A full-length cDNA of cotton aphid vesicle-fusing ATPase was amplified by PCR using an oligonucleotide comprising the nucleotide sequence of SEQ ID NO: 3 and an oligonucleotide comprising the nucleotide sequence of SEQ ID NO: 4, which are primers specific for the gene, and La-Taq polymerase (manufactured by Takara Bio) according to the manufacturer's procedure. First-strand cDNA, prepared as described above, was used as template. The PCR conditions used were those for touchdown PCR as follows: an initial denaturation at 94°C for 10 minutes; followed by 10 cycles of touchdown-PCR, one cycle being 94°C for 20 seconds, 60°C for 20 seconds with a decrease of 1°C per cycle, and 72°C for 2 minutes; followed by 25 cycles of PCR, one cycle being 94°C for 20 seconds, 50°C for 20 seconds, and 72°C for 3 minutes; and followed by 72°C for 7 minutes. The resulting PCR products were analyzed and purified by agarose gel electrophoresis to obtain 2421bp of DNA comprising the nucleotide sequence of SEQ ID NO. 2. The obtained DNA was cloned into the pCR4-TOPO vector (Invitrogen). An amino acid sequence presumed from the nucleotide sequence was the amino acid sequence of SEQ ID NO: 1.

Example 3 (Isolation of German cockroach vesicle-fusing ATPase Gene)

For isolation of homologues of vesicle-fusing ATPase gene from other insect species such as German cockroach (*Blatella germanic*), degenerate primers are designed using Codehop program (publicly accessible on the website of Blocks Protein Analysis Server operated within the Fred Hutchinson Cancer Research Center at http://blocks.fhcrc.org/blocks/codehop.html), and based on the sequence of the aforementioned cotton aphid-derived vesicle-fusing ATPase gene and the previously-known nucleotide sequences of D. melanogaster (NCBI accession number NM_080138). Manduca sexta (NCBI accession number AF118384), Helicoverpa zea (NCBI accession number AY220909), Anopheles gambiae (NCBI accession number XM_307148), and the like.

Partial sequences of a homologue of vesicle-fusing ATPase gene of a selected insect species are amplified by a series of PCR using first-strand cDNA derived_from the insect species as a template. Herein, the first-strand cDNA as a template is prepared by the aforementioned method using Superscript III. Amplification by PCR is performed using a set of degenerate primers as a forward primer and a reverse primer as well as Amplitaq Gold (manufactured by Applied Biosystems) according to the manufacturer's procedure annexed to the reagent. The PCR conditions are those for touchdown PCR as follows: an initial denaturation at 94° C for 10 minutes: followed by 10 cycles of touchdown-PCR, one cycle being 94°C for 30 seconds, 60°C for 1 minute with a decrease of 1°C per cycle, and 72°C for 1 minute and 30 seconds; followed by 25 cycles of PCR, one cycle being 94°C for 30 seconds, 50°C for 1 minute, and 72°C for 1 minute and 30 seconds; and followed by 72°C for 7 minutes. The PCR product is analyzed and purified by agarose gel electrophoresis to obtain DNA of interest. Further, the obtained DNA is cloned into the pCR4-TOPO vector (manufactured by Invitrogen), and sequenced.

Thus, partial sequence of a vesicle-fusing ATPase gene of Blatella germanica is obtained.

Then, primers specific for the resulting partial sequences of the insect homologue of vesicle-fusing ATPase gene are designed, and 3'RACE PCR or 5'RACE PCR is performed in order to obtain a full-length sequence of the gene. 3'and 5'RACE PCRs are performed employing first-strand cDNA prepared from the insect total DNA as a template and using SMART PCR cDNA Synthesis Kit (manufactured by Clontech) according to the manufacturer's instructions annexed to the kit.

In 3'RACE and 5'RACE reaction, universal primer mix (UPM) contained in SMART PCR cDNA Synthesis Kit is used in combination with a forward primer or a reverse primer which is specific for the sequence of interest. The PCR conditions are those for touchdown PCR as follows: an initial denaturation at 94°C for 10 minutes; followed by 10 cycles of touchdown-PCR, one cycle being 94°C for 30 seconds, 60°C for 1 minute with a decrease of 1°C per cycle, and 72°C for 2 minutes; followed by 25 cycles of PCR, one cycle being 94°C for 30 seconds, 50°C for 1 minute, and 72°C for 2 minutes; and followed by 72°C for 7 minutes. The resulting PCR product is analyzed and purified by agarose gel electrophoresis to obtain DNA of interest. Further, the obtained DNA is cloned into the pCR4-TOPO vector (manufactured by Invitrogen), and sequenced.

When a distinct amplification product is not obtained by the first-round PCR, nested PCR is performed using the first-round PCR product as a template. As primers, NLTP primer contained in SMART PCR cDNA Synthesis Kit is used in combination with a specific forward primer or a specific reverse primer which is designed to bind internal sequence of the first-round PCR product of interest. The PCR conditions used were those for touchdown PCR as follows: an initial denaturation at 94° C for 10 minutes; followed by 10 cycles of touchdown-PCR, one cycle being 94°C for 30 seconds, 60°C for 30 seconds with a decrease of 1°C per cycle, and 72°C for 2 minutes: followed by 25 cycles of PCR, one cycle being 94°C for 30 seconds, 50°C for 1 minute, and 72°C for 2 minutes; and followed by 72°C for 7 minutes. The resulting PCR product was analyzed and purified by agarose gel electrophoresis to obtain DNA of interest. Further, the obtained DNA is cloned into the pCR4-TOPO vector (manufactured by Invitrogen), and sequenced.

The above sequencing results reveal 5'-terminal sequence and 3'-terminal sequence, each encoding N-terminal region and C-terminal region of the insect vesicle-fusing ATPase, respectively.

Example 4 (Construction of recombinant plasmid)

A vesicle-fusing ATPase gene fragment to be cloned into a vector-for expression in E. coli was amplified by PCR using an oligonucleotide comprising the nucleotide sequence of SEQ ID NO: 5 which was a primer specific to the gene sequence; an oligonucleotide comprising the nucleotide sequence of SEQ ID NO: 6, which was a primer specific to the gene sequence and to which XhoI restriction site was added; and PfuUltra High Fidelity polymerase (manufactured by Stratagene) in accordance with the instructions attached to the polymerase. Full-length cDNA of the cotton aphid vesicle-fusing ATPase obtained in Example 2 was used as a template. The PCR conditions used were as follows: an initial denaturation at 94° C for 5 minutes; followed by 40 cycles of PCR, one cycle being 94°C for 20 seconds, 60°C for 20 seconds, and 72°C for 2 minutes; followed by 72°C for 7 minutes.

The resulting PCR product was purified using the Qiaquick PCR Purification Kit (manufactured by Qiagen) in accordance with the instruction attached to the kit. The DNA fragment after the purification was digested with XhoI.

The Blunt/XhoI DNA fragment of cotton aphid vesicle-fusing ATPase gene was analyzed by an agarose gel electrophoresis, isolated, purified and ligated into the EcoRV/XhoI cloning sites of the E. coli expression vector pET41b (+) (manufactured by Novagen). The obtained vector was called pGAY008.

Translation of the recombinant vesicle-fusing ATPase togther with two His-tags and one GST-tag provided a recombinant protein of 769 amino acids.

Following the procedures described in Qiagen Plasmid Purification Handbook, pGAY008 was prepared using a Qiafilter Plasmid Maxiprep (Qiagen).

Example 5 (Preparation of recombinant E. coli)

Competent cells of E. coli BL21 (DE3) (manufactured by Invitrogen) were transformed following the manufacturers instruction, using $1\mu1$ of pGAY008 at a concentration of Ing/$\mu$l. Colonies of the transformed E. coli were grown on LB agar plates containing 50 mg/L Kanamycin (manufactured by Sigma) at 37°C overnight.

Example 6 (Expression of vesicle-fusing ATPase in E. coli)

E. coli BL21 (DE3) transformed with pGAY008 was rotary cultured overnight in LB-medium containing 50 mg/L of Kanamycin (manufactured by Sigma) at 37°C, 250 rpm. In the next morning, the culture was diluted to 1/25 in LB-medium containing 50 mg/L of Kanamycin, and grown at 37°C, 250rpm. When the culture reached an OD of 0.8 at 600 nm, IPTG was added to the culture until a final concentration of $10\mu$M. The culture was incubated at 25°C, 60rpm for 4 days to produce the recombinant vesicle-fusing ATPase in the E. coli. After induction and expression, the culture was centrifused at 7,000 rpm for 10 minutes to harvest the E. coli. The supernatant was discarded, and the remaining E. coli was flash frozen in liquid nitrogen and stored at -80°C until usage.

Example 7 (Purification of vesicle-fusing ATPase)

The cotton aphid vesicle-fusing ATPase was cloned in pET41(b+) in frame with a N-terminal GST (glutathione S-transferase)-tag and His-tag and a C-terminal His-tag. The recombinant vesicle-fusing ATPase protein was purified through two following purification procedures: the first one utilizing a His-tag, and the second one utilizing a GST-tag.

(1) Preparation of crude extract

The frozen cell pellets of induced E. coli BL21(DE3) cell were resuspended in 30 ml of breaking buffer(100mM Hepes/KOH pH 7.5, 500 mM KCl, 5mM $MgCl_2$ , 2 mM 2-mercaptoethanol, 5 mM ATP, 0.5 mM, Pefablock, 10 $\mu$g/$\mu$l Leupeptin, 1 $\mu$M Pepstatin A), and subsequently lysed in breaking buffer by using French press (manufactured by Thermo Spectronic) . The pressure was maintained at 1300 to 1500 psi during the procedure of breaking of the cells. The French pressed solution was centrifuged for 60 minutes at 14,000 rpm at 2°C to collect a supernatant. The collected supernatant was filtered through a 0.45$\mu$m filter and kept on ice.

(2) Purification utilizing His-tag

The recombinant protein has been going through a first purification utilizing metal affinity chromatography, using either the HiTrap Chelating HP (Amersham biosciences) or HisTrap HP (Amersham biosciences) columns, according to the instructions of the manufacturer (Amersham biosciences). For larger scale purifications, a XK-16/20 column (Amersham biosciences) was used, the column being filled with Chelating Fast Flow Sepharose (Amersham biosciences). The purification procedure was undertaken on the AEKTA-FPLC (Amersham biosciences) .

Hitrap, HisTrap, AX-16/20 affinity columns (manufactured by Amersham biosciences) have been prepared according to the manufacturer's protocol (Amersham biosciences). Buffer A, the binding buffer, was made of 20 mM Hepes pH 7.0, 200 mM KCl, 1mM $MgCl_2$ , 2 mM 2 -mercaptoethanol, 0.5 mM ATP, and 10% glycerol. Buffer B, the elution buffer, was made of 20mM Hepes pH 7.0, 200 mM KCl, 1mM $MgCl_2$, 2 mM 2-mercaptoethanol, 0.5 mM ATP, 500 mM imidazole, and 10% glycerol.

The purification of cotton aphid vesicle-fusing ATPase utilizing a His-tag was performed as the following procedure:

(i) sample injection;
(ii) washing out unbound sample with 5 column volumes (CV) of 85% buffer A/15% buffer B;
(iii) washing for 15 CV with 40% buffer B (200 mM imidazole);
(iv) elusion of purified protein with 15 CV of 70% buffer B (350 mM imidazole); and
(v) washing the column with 5 CV of 100 % buffer B (500 mM imidazole) .

The fractions obtained from the elution with 30% buffer A/70% buffer B were pooled and stored on ice until initiation of the purification utilizing GST-tag.

(3) Purification utilizing GST-tag

The pooled His-purified fractions from the first purification have been purified a second time over a GSTrap FF column ( Amersham biosciences), according to the manufacturers manual. The purification procedure was undertaken on the AEKTA-FPLC (Amersham biosciences).

Buffer A, the binding buffer, was made of 20 mM Hepes pH 7.0, 200 mM KCl, 1mM $MgCl_2$, 2 mM 2-mercaptoethanol and 10% glycerol. Buffer B, the elution buffer, was made of 50 mM Tris-HCl, 10 mM reduced glutathione, 2 mM 2-mercaptoethanol and 10% glycerol. After addition of glutathione, the pH was adjusted to pH7.5 with KOH.

The purification of cotton aphid vesicle-fusing ATPase utilizing a GST-tag was performed as the following procedure:

(i) sample injection;
(ii) washing out unbound sample with 20 column volumes (CV) of 100%buffer A; and
(iii) elution of purified protein with 30 CV of 100% buffer B.

The obtained elution fractions were analysed to verify presence of the recombinant cotton aphid vesicle-fusing ATPase protein by means of standard techniques of Coomassie stained SDS-PAGE and western blot. An 8% polyacrylamide gel was used for optimal gel electrophoresis resolution of the expressed vesicle-fusing ATPase protein of 86kDa.

The following staining solution and destain solution were used for Coomassie staining of polyacrylamide gel. Staining solution was made of 1g/l Coomassie Brilliant blue R, 50 (v/v)% Methanol, 12 (v/v)% Acetic acid and 38 (v/v) % distilled water. After mixing, the solution was filtered to get out unsoluble Brilliant blue R dye. Detain solution was made of 25 (v/v)% Methanol, 10 (v/v)% Acetic acid and 65 (v/v)% distilled water.

For western blot analysis, an anti - His(H15) sc-803 rabbit polyclonal IgG antibody (tebubio) was used as primary antibody at a 1:500 dilution. The secondary antibody was a goat anti-rabbit-HRP (Pierce) at a dilution of 1:10000.

After analysis of the polyacrylamide gels by SDS-PAGE and Western blotting, the fractions of interest were pooled and the protein concentration was determined. Protein Concentration was determined by Bradford method with the Bradford Bio-Rad protein assay (Bio-Rad) using Pre-diluted Protein Assay Standards (Pierce): Bovine Serum Albumin Fraction V Set according to the manufacturer.'s protocol. The pooled fractions were then distributed into several aliquots and immediately flash-frozen in liquid nitrogen and stored at -80 °C.

Example 8 (Selection of compounds inhibiting vesicle-fusing ATPase activity)

Selection of a compound which modulates a vesicle-fusing ATPase activity was performed in a system for measuring and evaluating the vesicle-fusing ATPase activity, the activity being modulated by adding a test compound to an in vitro reaction system using the cotton aphid vesicle-fusing ATPase prepared in Example 7.

As for a measurement of the cotton aphid vesicle-fusing ATPase activity, after the enzymatic reaction of vesicle-fusing ATPase using ATP as a substrate, an amount of ATP that was not hydrolyzed was measured as the amount of luminescence of luciferase using Kinase-Glo™ Luminescent Kinase Assay (manufactured by Promega) , according to a method described in the instruction attached to the kit. The vesicle-fusing ATPase activity was calculated from the amount of luminescence.

For measuring the activity, the activity of the aphid vesicle-fusing ATPase was measured when a test compound dissolved in DMSO was contained to a final concentration of 10 μM. In addition, the activity of the aphid vesicle-fusing ATPase was measured when DMSO was contained in place of a test compound. Then, a ratio (%) of a measured value of the activity of the aphid vesicle-fusing ATPase when a test compound dissolved in DMSO was contained, relative to a measured value of the activity of aphid vesicle-fusing ATPase when DMSO was contained in place of the test compound was calculated, and a value obtained by subtracting the calculated value from 100% was adopted as an inhibition degree (%). The results in each test compound are shown in Table 4 in Example 9 together with results of Example 9.

The activity of aphid vesicle-fusing ATPase was measured when a test compound dissolved in DMSO was contained to a final concentration of each concentration of 100 μM, 30 μM, 10 μM, 3 μM, 1 μM, 0.3 μM, 0.1 μM or 0.03 μM. $IC_{50}$ (μM) was calculated from the result of each concentration at each test compound using a concentration-dependent test analyzing software XL fit (manufactured by idbs) . The results are shown in Table 5 in Example 10 together with results of Example 9.

Example 9 (Pesticidal activity test)

A sterilized artificial feed having the following composition (Table 3) was prepared. Then, according to the same manner as that of the method described in Handbook of Insect Rearing Vol.1 (Elsevier Science Publisers 1985) pp. 35 to pp. 36 except that a test compound dissolved in DMSO to a final concentration of 50 ppm was added at 0.5 % volume of the artificial feed, and components were mixed, Aphis gossypii was reared. Six days after rearing, the number of surviving Aphis gossypii was investigated, and an entity exhibiting a significant controlling value (e.g. controlling value of 30% or more) was determined to have pesticidal activity by obtaining a controlling value by the following equation.

$$\text{Controlling value (\%)} = \{1 - (Cb \times Tai)/(Cai \times Tb)\} \times 100$$

Letters in the equation represent the following meanings.

Cb: Number of surviving worms before treatment in non-treated section
Cai: Number of surviving worms at observation in non-treated section
Tb: Number of surviving worms before treatment in treated section Tai: Number of surviving worms at observation in treated section

Results are shown in Table 4 in Example 9 together with results of Example 8.

Table 3

| Amino acid | (mg/100 ml) | Vitamins | (mg/100 ml) |
|---|---|---|---|
| L-alanine | 100.0 | Ascorbic acid | 100.0 |
| L-arginine | 275.0 | Biotin | 0.1 |
| L-asparagine | 550.0 | Calcium pantothenate | 5.0 |
| L-aspartic acid | 140.0 | Choline chloride | 50.0 |
| L-cysteine (hydrochloride) | 40.0 | Inositol | 50.0 |
| L-glutamic acid | 140.0 | Nicotinic acid | 10.0 |
| L-glutamine | 150.0 | Thiamine | 2.5 |
| L-glycine | 80.0 | | |
| L-histidine | 80.0 | Others | (mg/100 ml) |
| L-isoleucine | 80.0 | Sucrose | 12500.0 |
| L-leucine | 80.0 | Dipotassium hydrogen phosphate | 1500.0 |
| L-lysine (hydrochloride) | 120.0 | Magnesium sulfate | 123.0 |
| L-methionine | 80.0 | Cupric chloride | 0.2 |
| L-phenylalanine | 40.0 | Ferric chloride | 11.0 |

(continued)

| Amino acid | (mg/100 ml) | Vitamins | (mg/100 ml) |
|---|---|---|---|
| L-proline | 80.0 | Manganese chloride | 0.4 |
| L-serine | 80.0 | Zinc sulfate (anhydrous) | 0.8 |
| L-threonine | 140.0 | | |
| L-tryptophan | 80.0 | Adjusted to pH 6.8 | |
| L-tyrosine | 40.0 | | |
| L-valine | 80.0 | | |

Table 4

| Compound | Result of Example 8 | Result of Example 9 |
|---|---|---|
| | Activity of inhibiting vesicle-fusing ATPase activity (inhibition degree (%) at 10 μM addition) | Determination result of pesticidal activity |
| Suramin | 100 | Presence of pesticidal activity |
| Calmidazolium Chloride | 80 | Presence of pesticidal activity |
| NPC15437 dihydrochloride | 35 | Presence of pesticidal activity |

Example 10 (Pesticidal activity test)

According to the same manner as that of Example 9 , pesticidal activity test was performed, and results are shown in Table 5 in Example 10 together with results of Example 8.

Table 5

| Compound | Result of Example 8 | Result of Example 10 |
|---|---|---|
| | Activity of inhibiting vesicle-fusing ATPase activity (IC50, μM) | Determination result of pesticidal activity |
| Suramin | 1.9 | Presence of pesticidal activity |
| Calmidazolium Chloride | 4.7 | Presence of pesticidal activity |
| NPC15437 dihydrochloride | 19.0 | Presence of pesticidal activity |
| Hypocrellin B | > 100 | Absence of pesticidal activity |
| (±)-Blebbistatin | > 100 | Absence of pesticidal activity |

Industrial Applicability

According to the present invention, it becomes possible to provide a more target-based approach of screening agricultural chemicals, whereby compounds are screened against a specific target with intent of chemically interfering with the target site to control insects or other pest organism.

Free Text in Sequence Listing

SEQ ID NO: 3
Designed oligonucleotide primer for PCR
SEQ ID NO: 4
Designed oligonucleotide primer for PCR

SEQ ID NO: 5
Designed oligonucleotide primer for PCR
SEQ ID NO: 6
Designed oligonucleotide primer for PCR

Furthermore, the present invention relates to the following items:

1. An agent that modulates physiological condition of pests, wherein said agent has an ability to modulate the activity of an insect vesicle-fusing ATPase (EC. 3.6.4.6).

2. An agent according to item 1, wherein said vesicle-fusing ATPase is a cotton aphid vesicle-fusing ATPase :

3. An agent according to item. 1, wherein said agent is a pesticidal agent.

4. An agent according to item 1, wherein said ability to modulate the activity of an insect vesicle-fusing ATPase is an ability to inhibit a reaction of the insect vesicle-fusing ATPase with ATP.

5. A pesticidal agent which comprises a substance that has an ability to modulate the activity of an insect vesicle-fusing ATPase or an agriculturally acceptable salt of the substance as an active ingredient.

6. A pesticidal agent according to item 5, wherein said substance has an ability to inhibit a reaction of the insect vesicle-fusing ATPase with ATP.

7. A pesticidal agent according to item 6, wherein said substance has an ability to inhibit the reaction of the insect vesicle-fusing ATPase with ATP in a cell-free system, wherein in the presence of said substance of 10 micro M or more the activity of said vesicle-fusing ATPase is lower than that in the absence of said substance.

8. A pesticidal agent according to item 6, wherein said substance has an ability to inhibit a reaction of the insect vesicle-fusing ATPase with ATP in a cell-free system with an IC50 of 100 micro M or less.

9. A method for assaying pesticidal activity of a test substance, which comprises:

   (1) a first step of measuring the activity of a vesicle-fusing ATPase selected from the following group A in a reaction system in which said vesicle-fusing ATPase contacts with a test substance; and
   (2) a second step of evaluating the pesticidal activity of said test substance based on the difference obtained by comparing the activity measured in the first step with the activity of a control:

      <group

         (a) a protein comprising the amino acid sequence of SEQ ID NO: 1;
         (b) a protein comprising an amino acid sequence with deletion, addition or substitution of one or more amino acids in the amino acid sequence of SEQ ID NO: 1, wherein said protein has vesicle-fusing ATPase activity;
         (c) a protein comprising an amino acid sequence that has sequence identity of 75% or more to the amino acid sequence of SEQ ID NO: 1. wherein said protein has vesicle-fusing ATPase activity;
         (d) a protein comprising the amino acid sequence encoded by the nucleotide sequence of SEQ ID NO: 2;
         (e) a protein comprising an amino acid sequence encoded by a nucleotide sequence that has sequence identity of 75% or more to the nucleotide sequence of SEQ ID NO: 2, wherein said,protein has vesicle-fusing ATPase activity;
         (f) a protein comprising an amino acid sequence encoded by a polynucleotide, wherein said polynucleotide hybridizes under a stringent condition to a polynucleotide comprising a nucleotide, sequence complementary to the nucleotide sequence of SEQ ID NO: 2, and wherein said protein has vesicle-fusing ATPase activity;
         (g) a protein comprising an amino acid sequence of an insect vesicle-fusing ATPase; and
         (h) a protein comprising an amino acid sequence of a cotton aphid vesicle-fusing ATPase.

10. A method for screening a pesticidal substance, which comprises selecting a substance having the pesticidal activity that is evaluated by the method of item 9.

11. A pesticidal agent which comprises a substance selected by the method of item 10 or agriculturally acceptable salts thereof as an active ingredient.

12. A method for controlling pests which comprises applying an effective amount of the pesticidal agent of item 5, 6,7,8 or 11 to the pest, habitat of the pest or plant to be protected from the pest.

13. A method for controlling pests which comprises:

identifying a substance having the pesticidal activity that is evaluated by the method of item 9, and contacting the pest with the identified pesticidal substance.

14. An insect vesicle-fusing ATPase comprising an amino acid sequence selected from the following group B:

<group B>

(a) the amino acid sequence of SEQ ID NO: 1;
(b) an amino acid sequence with deletion, addition or substitution of one or more amino acids in the amino acid sequence of SEQ ID NO: 1, wherein said amino acid sequence has vesicle-fusing ATPase activity;
(c) an amino acid sequence that has sequence identity of 75% or more to the amino acid sequence of SEQ ID NO: 1, wherein said amino acid sequence has vesicle-fusing ATPase activity;
(d) the amino acid sequence encoded by the nucleotide sequence of SEQ ID NO: 2;
(e) an amino acid sequence encoded by a nucleotide sequence that has sequence identity of 75 % or more to the nucleotide sequence of SEQ ID NO: 2 , wherein said amino acid sequence has vesicle-fusing ATPase activity;
(f) an amino acid sequence encoded by a polynucleotide, wherein said polynucleotide hybridizes under a stringent condition to a polynucleotide comprising a nucleotide sequence complementary to the nucleotide sequence of SEQ ID NO 2, wherein said amino acid sequence has vesicle-fusing ATPase activity; and
(g) an amino acid sequence of a cotton aphid vesicle-fusing ATPase.

15. Use of an insect vesicle-fusing ATPase as a reagent that provides an indicator to evaluate pesticidal activity.

16. Use of an inspect vesicle-fusing ATPase of item 14 as a reagent that provides an indicator to evaluate pesticidal activity.

17. A polynucleotides which comprises a nucleotide sequence encoding an amino acid sequence of a vesicle-fusing ATPase of item 14.

18. A polynucleotides according to item 17, which comprises the nucleotide sequence of SEQ ID NO: 2.

19. A polynucleotide which comprises a nucleotide sequence complementary to a nucleotide sequence of a polynucleotide of item 17 or 18.

20. A polynucleotide which comprises:

a partial nucleotide sequence of a polynucleotide of item 17 or 18; or
a nucleotide sequence complementary to said partial nucleotide sequence.

21. A polynucleotide according to item 20, which comprises a nucleotide sequence of SEQ ID NO: 3 or 4.

22. A method for obtaining a polynucleotide comprising a nucleotide sequence encoding an amino acid sequence of a vesicle-fusing ATPase, which comprises:

amplifying a desired polynucleotide by polymerase chain reaction using as a primer a polynucleotide of item 20 or 21;
identifying the desired polynucleotide amplified; and
recovering the identified polynucleotide.

23. A method for obtaining a polynucleotides comprising a nucleotide sequence encoding an amino acid sequence of a vesicle-fusing ATPase, which comprises:

detecting a desired polynucleotide by hybridization using as a probe a polynucleotide of item 19, 20 or 21;
identifying the desired polynucleotide detected; and
recovering the identified polynucleotide.

24. A circular polynucleotide comprising a nucleotide sequence of a polynucleotide of item 17 or 18, wherein said nucleotide sequence is operably linked to a bacteriophage promoter.

25. A circular polynucleotide according to item 24, wherein said promoter is a T7 RNA polymerase gene promoter.

26. A circular polynucleotide according to item 24 or 25, wherein said polynucleotide comprises a replication origin for autonomous replication in a host cell.

27. A method for producing a circular polynucleotide, which comprises ligating a polynucleotide of item 17 or 18 into a vector.

28. A transformant in which a polynucleotide of item 17 or 18 is introduced.

29. A transformant according to item 28, wherein said transformant is a transformed E. coli.

30. A method for producing a transformant, which comprises introducing a polynucleotide of item 17 or 18 into a host cell.

31. A method for producing a vesicle-fusing ATPase, which comprises a step of culturing the transformant of item 28 or 29 and recovering a produced vesicle-fusing ATPase.

32. Use of a vesicle-fusing ATPase of item 14 or a polynucleotide of any one of items 17 to 21 as a research tool.

33. Use according to item 32, wherein the research tool is an experimental tool for screening a pesticidal substance.

34. A system which comprises:

a means to input, store and manage a data information of an ability of test substances, wherein said ability is an ability to modulate the activity of an insect vesicle-fusing ATPase;
a means to query and retrieve the data information based on a desired criterion; and
a means to display and output the result which is queried and retrieved.

**Claims**

1. A method for assaying pesticidal activity of a test substance, which comprises:

   (1) a first step of measuring the activity of a vesicle-fusing ATPase selected from the following group A in a reaction system in which said vesicle-fusing ATPase contacts with a test substance; and
   (2) a second step of evaluating the pesticidal activity of said test substance based on the difference obtained by comparing the activity measured in the first step with the activity of a control:

      <group A>

      (a) a protein comprising the amino acid sequence of SEQ ID NO: 1;
      (b) a protein comprising an amino acid sequence with deletion; addition or substitution of one or more amino acids in the amino acid sequence of SEQ ID NO: 1, wherein said protein has vesicle-fusing ATPase activity;
      (c) a protein comprising an amino acid sequence that has sequence identity of 75% or more to the amino acid sequence of SEQ ill NO: 1, wherein said protein has vesicle-fusing ATPase activity;
      (d) a protein comprising the amino acid sequence encoded by the nucleotide sequence of SEQ ID NO: 2;
      (e) a protein comprising an amino acid sequence encoded by a nucleotide sequence that has sequence identity of 75% or more to the nucleotide sequence of SEQ ID NO: 2, wherein said protein has vesicle-fusing ATPase activity;

(f) a protein comprising an amino acid sequence encoded by a polynucleotide, wherein said polynucleotide hybridizes under a stringent condition to a polynucleotide comprising a nucleotide sequence complementary to the nucleotide sequence of SEQ ID NO: 2, and wherein said protein has vesicle-fusing ATPase activity;

(g) a protein comprising an amino acid sequence of an insect vesicle-fusing ATPase; and

(h) a protein comprising an amino acid sequence of a cotton aphid vesicle-fusing ATPase.

2. A method for screening a pesticidal substance, which comprises selecting a substance having the pesticidal activity that is evaluated by the method of claim 1.

3. An insect vesicle-fusing ATPase comprising an amino acid sequence selected from the following group B:

<group

(a) the amino acid sequence of SEQ ID NO: 1;

(b) an amino acid sequence with deletion, addition or substitution of one or more amino acids in the amino acid sequence of SEQ ID NO: 1, wherein said amino acid sequence has vesicle-fusing ATPase activity;

(c) an amino acid sequence that has sequence identity of 75% or more to the amino acid sequence of SEQ ID NO: 1, wherein said amino acid sequence has vesicle-fusing ATPase activity;

(d) the amino acid sequence encoded by the nucleotide sequence of SEQ ID NO: 2;

(e) an amino acid sequence encoded by a nucleotide sequence that has sequence identity of 75% or more to the nucleotide sequence of SEQ ID NO: 2, wherein said amino acid sequence has vesicle-fusing ATPase activity;

(f) an amino acid sequence encoded by a polynucleotide, wherein said polynucleotide hybridizes under a stringent condition to a polynucleotide comprising a nucleotide sequence complementary to the nucleotide sequence of SEQ ID NO: 2, wherein said amino acid sequence has vesicle-fusing ATPase activity; and;

(g) an amino acid sequence of a cotton aphid vesicle-fusing ATPase.

4. Use of an insect vesicle-fusing ATPase or an insect vesicle-fusing ATPase of claim 3 as a reagent that provides an indicator to evaluate pesticidal activity.

5. A polynucleotide which comprises:

a nucleotide sequence encoding an amino acid sequence of a vesicle-fusing ATPase of claim 3; or
the nucleotide sequence of SEQ ID NO: 2.

6. A polynucleotide which comprises a nucleotide sequence complementary to a nucleotide sequence of a polynucleotide of claim 5.

7. A polynucleotide which comprises:

a partial nucleotide sequence of a polynucleotide of claim 5;
a nucleotide sequence complementary to said partial nucleotide sequence;
the nucleotide sequence of SEQ ID NO: 3; or
the nucleotide sequence of SEQ ID NO: 4.

8. A method for obtaining a polynucleotide comprising a nucleotide sequence encoding an amino acid sequence of a vesicle-fusing ATPase, which comprises:

(a-i) amplifying a desired polynucleotide by polymerase chain reaction using as a primer a polynucleotide of claim 7,
(a-ii) identifying the desired polynucleotide amplified, and
(a-iii) recovering the identified polynucleotide; or
(b-i) detecting a desired polynucleotide by hybridization using as a probe a polynucleotide of claim 6 or 7,
(b-ii) identifying the desired polynucleotide detected, and
(b-iii) recovering the identified polynucleotide.

9. A circular polynucleotide comprising a nucleotide sequence of a polynucleotide of claim 5, wherein said nucleotide sequence is operably linked to a bacteriophage promoter or a T7 RNA polymerase gene promoter; or wherein said polynucleotide preferably comprises a replication origin for autonomous replication in a host cell.

10. A method for producing a circular polynucleotide, which comprises ligating a polynucleotide of claim 5 into a vector.

11. A transformant in which a polynucleotide of claim 5 is introduced.

12. A transformant according to claim 11, wherein said transformant is a transformed E.coli.

13. A method for producing a transformant, which comprises introducing a polynucleotide of claim 5 into a host cell.

14. A method for producing a vesicle-fusing ATPase, which comprises a step of culturing the transformant of claim 11 or 12 and recovering a produced vesicle-fusing ATPase.

15. Use of a vesicle-fusing ATPase of claim 3 or a polynucleotide of any one of claims 5 to 7 as a research tool, preferably as an experimental tool for screening a pesticidal substance.

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- WO 0009682 A

## Non-patent literature cited in the description

- **CHENE.** *Nature Reviews Drug Discovery,* 2002, vol. 1, 665-673
- **HANLEY et al.** *Neuron,* 2002, vol. 34, 53-67
- **MAY et al.** *J. Biol.Chem.,* 2001, vol. 276, 21991-21994
- **GOLBY et al.** *Genetics,* 2001, vol. 158, 265-278
- **CUI ; XU.** *Peptides,* 2006, vol. 27 (6), 1226-1234
- **CUI et al.** *Insect Biochem Mol Biol.,* 2006, vol. 36 (7), 603-9
- **LITTLETON et al.** *Proc. Natl. Acad. Sci. USA,* 2001, vol. 98, 12233-12238
- **TAGAYA et al.** *J Biol Chem,* 1993, vol. 268, 2662-2666
- **MUELLER et al.** *Nature Cell Biol,* 1999, vol. 1, 335-340
- **PETERS et al.** *EMBO J,* 1990, vol. 9, 1757-1767
- **LANZETTA et al.** *Analytical Biochemistry,* 1979, vol. 100, 95-97
- **LILL et al.** *Cell,* 1990, vol. 60, 271-280
- **CHENE.** *Nature reviews,* 2002, vol. 1, 665
- **BEUKERS et al.** *aunyn-Schmied. Arch. Pharmacol.,* 1995, vol. 351, 523-528
- **STEEL G. J. et al.** *Biochemistry,* 1999, vol. 38, 7764-7772
- **MORGAN A. et al.** *JBC,* 1994, vol. 269 (7), 29347-29350
- Handbook of Insect Rearing. Elsevier Science Publisers, vol. 1, 35-36
- *Nucleic Acids Res.,* 1984, vol. 12, 9441-9456
- *Method in Molecular Biology,* 1994, 97-112
- **PEARSON ; LIPMAN.** *Proc. Natl. Acad. Sci. USA,* 1988, vol. 4, 2444-2448
- **ALTSCHUL et al.** *Journal of Molecular Biology,* 1990, vol. 215, 403-410
- **THOMPSON ; HIGGINS ; GIBSON.** *Nucleic Acid Research,* 1994, vol. 22, 4673-4680
- **LIPMAN, D. J. ; PEARSON, W.R.** *Science,* 1985, vol. 227, 1435-1441
- **SAMBROOK J. ; FRISCH E. F. ; MANIATIS T.** Molecular Cloning. Cold Spring Harbor Laboratory press
- Molecular Biology. John Wiley & Sons, 1989, 6.3.1-6.3.6
- Molecular Cloning: A Laboratory Manual. Cold Spring Harbor Laboratory Press, 1989
- Current Protocols In Molecular Biology. John Wiley & Sons,Inc, 1987
- **MAXAM,A.M ; W.GILBERT.** *Proc.Natl.Acad.Sci.USA,* 1977, vol. 74, 560
- **SANGER,F. ; A.R.COULSON.** *J.Mol.Biol.,* 1975, vol. 94, 441
- **SANGER,F ; NICKLEN ; A.R.COULSON.** *Proc.Natl.Acad.Sci.USA,* 1977, vol. 74, 5463
- **SANGER,F. ; A.R.COULSON.** *J.Mol,Biol.,* 1975, vol. 94, 441
- **SANGER,F ; NICKLEN ; A.R.COULSON.** *Proc.Natl.Acad.Sci.USA,* 1977, vol. 74, 5463
- Handbook of Insect Rearing. Elsevier Science Publisers, 1985, vol. 1, 35-36